# EUROPÄISCHE PATENTSCHRIFT

(11) **EP 3 878 346 B1**
(45) Veröffentlichungstag und Bekanntmachung des Hinweises auf die Patenterteilung: **21.08.2024**
(21) Anmeldenummer: 21161015.9
(22) Anmeldetag: 05.03.2021
(51) Int. Cl.: A61B 1/00, G02B 6/00, G02B 23/24, A61B 1/05, A61B 1/06, A61B 1/313

(54) **ENDOSKOP UND EINWEG-ENDOSKOPSYSTEM**
ENDOSCOPE AND DISPOSABLE ENDOSCOPE SYSTEM
ENDOSCOPE ET SYSTÈME D'ENDOSCOPE JETABLE

(30) Priorität: 13.03.2020 DE 102020106915
(43) Veröffentlichungstag der Anmeldung: 15.09.2021
(60) Teilanmeldung: 24187803.2
(73) Patentinhaber: SCHOTT AG, 55122 Mainz (DE); SCHOTT Corporation, Rye Brook, NY 10573 (US)
(72) Erfinder: SCHULTHEIS, Bernd, 55270 Schwabenheim (DE); CRAMER, Martin, 65187 Wiesbaden (DE); RUSSERT, Hubertus, 55270 Jugenheim (DE); WERNER, Holger, 60596 Frankfurt am Main (DE); MEINL, Jürgen, 65329 Hohenstein-Holzhausen (DE); GRIMM, Jonas, 65307 Bad Schwalbach (DE); KEIPER, Oliver, 65510 Hünstetten (DE); ASTHEIMER, Steffen, 65510 Hünstetten (DE); BLEISINGER, Björn, 55499 Riesweiler (DE); SCHWEDLER, Christian, 55130 Mainz (DE); DUFFY, Robert F., Stafford Springs, CT 06076 (US)
(74) Vertreter: Blumbach · Zinngrebe Patentanwälte PartG mbB

(56) Entgegenhaltungen:
- DE-A1- 102007 026 234
- DE-A1- 102015 015 041
- DE-A1- 102018 107 523
- DE-B4- 102011 119 972
- DE-T2- 69 720 736
- US-A1- 2017 231 698

## Beschreibung

### Gebiet der Erfindung

Die Erfindung betrifft allgemein Endoskope und Endoskopsysteme, insbesondere auch Einweg-Endoskope und/oder Einweg-Endoskopsysteme.

### Hintergrund der Erfindung

Diagnose-, Operations- und/oder Therapiegeräte, wie beispielsweise Endoskope zur Diagnose, für minimalinvasive Eingriffe oder zur Therapie, sind als starre oder flexible Ausführungen bekannt und in der Literatur hinreichend beschrieben. Einweg-Endoskope, oder auch "disposable Endoscopes" genannt, werden heute zunehmend eingesetzt, um insbesondere die Patientensicherheit bei medizintechnische Untersuchungen, Therapien und/ oder minimal invasive Eingriffen zu erhöhen, in dem durch eine Einmal-Verwendung Kontaminationen verhindert werden. Zwar sind bisherige Endoskope dafür konzipiert, dass diese im Sinne der Medizintechnik aufbereitbar, das heißt reinigbar, sterilisierbar und vor allem autoklavierbar, sind.

Trotzdem kann es hier aufgrund falscher Anwendung der Aufbereitung bzw. ungünstigem Design derartiger Geräte vereinzelt vorkommen, dass nicht die erforderliche Keimzahlreduzierung erreicht wird, und damit Keime bei der nächsten Anwendung auf den Patienten übertragen werden können. Dies kann durch den Einsatz von derartigen Einweg-Endoskopen verhindert werden.

Ein weiterer Aspekt für den vermehrten Einsatz von Einweg-Endoskopen stellt auch eine Wirtschaftlichkeitsbetrachtung dar. Insbesondere der ordnungsgemäß und regelmäßig nach jeder Behandlung durchgeführte Aufbereitungsprozess erfordert inzwischen hohe Kosten beim praktizierenden Arzt oder in der Klinik. Zudem sind hohe Investitionen für Reinigungsgeräte, wie Thermodesinfektoren, und Autoklaviergeräten und/ oder Plasma-Sterilisationsgeräte erforderlich, so dass insgesamt der Einsatz derartiger Einweg-Endoskope gerechtfertigt ist.

Ein weiterer Vorteil ergibt sich daraus, dass derartige Einweg-Endoskope zum einen mobil als "hand-held"-Geräte einsetzbar sind und daher auch in der Notfall-Medizin, im Militär-Sanitätseinsatz oder auch in schwer zugänglichen Regionen, zum Beispiel auch bei Katastropheneinsätzen, einsetzbar sind, wo insbesondere keine Aufbereitungsmöglichkeiten zur Verfügung stehen.

Derartige Einweg-Endoskope, "Single-Use"-Endoskope oder "disposable Endoscopes", wie sie in der Literatur beschrieben sind, sind beispielhaft in folgenden Schriften beschrieben:
Die Schrift US 3581738 A1 offenbart ein Einweg-Endoskop, umfassend einen Körper aus synthetischem Harzmaterial mit einer im allgemeinen rohrförmigen Seitenwand, die ein Spekulum bildet, und ein einheitliches längliches Lichtleitelement, das in die Seitenwand eingebettet ist, wobei das Element aus einem Lichtleitmaterial gebildet ist, das mit einem transparenten Material mit einem Brechungsindex, der sich von dem des Lichtleitmaterials unterscheidet, überzogen ist, wobei der Körper aus zwei sich axial vom Endoskop geteilten Paarungshälften gebildet ist, wobei jede Hälfte ein Elementumschließungselement aufweist.

In der Schrift US 4964710 A1 wird ein starres Endoskop beschrieben, welches mit einem Objektivsystem, einer Okularlinse und einer Zwischenrelaislinse ausgestattet. Das Relaissystem ist ein Hybridsystem, das sowohl Kunststoff- als auch Glaselemente verwendet. Die Kunststoffelemente bestehen aus einer geradzahligen Anzahl (N) axial ausgerichteter Linsen, die jeweils eine Länge in der gleichen Größenordnung wie ihr Durchmesser aufweisen. Die Glaselemente sind eine ungeradzahlige Anzahl (N minus 1) von axial ausgerichteten Glas-Planzylindern, deren Stirnflächen poliert sind.

Die Schrift EP 1890173 A1 beschreibt eine Methode zur Herstellung eines Lichtleiters, wie er in derartigen Endoskopen einsetzbar ist. Dabei werden eine Vielzahl von optischen Fasern gebündelt und anschließend das Faserbündel an einem Teil eines Mundstücks geschnitten, das an einem Zwischenteil des Faserbündels befestigt ist. So wird das Faserbündel in ein erstes optisches Faserbündel und ein zweites optisches Faserbündel aufgeteilt. Die Teilungsflächen des ersten und zweiten optischen Faserbündels haben die gleichen Eigenschaften und Bedingungen, da die ersten und zweiten optischen Faserbündel aus dem Faserbündel gebildet sind, das durch Bündelung der gleichen optischen Fasern erhalten wird. Das erste optische Faserbündel ist in einem Einführungsabschnitt eines Endoskops montiert und das zweite optische Faserbündel ist in einem flexiblen Schlauch montiert, womit ein erster Lichtleiter in dem Einführungsabschnitt des Endoskops und ein zweiter Lichtleiter in dem flexiblen Schlauch ausgebildet sind. Dadurch entsteht eine trennbare Lichttransmissionsstrecke des Lichtleiters.

Da aufgrund ihrer Einmal-Verwendung derartige Endoskope unter einem hohen Kostendruck stehen, müssen die Baugruppen bzw. Komponenten kostenoptimiert herstellbar sein. Eine der Hauptkomponenten zur Bildgebung und Beleuchtung stellen Lichtleiter oder Bildleiter dar. Diese werden derzeit noch in vergleichsweise aufwendigen Prozessschritten montiert bzw. bearbeitet. Oft sind es komplexe mechanische Komponenten, teils kombiniert mit optischen Elementen, wie Linsen, die diese Licht- bzw. Bildleiter beinhalten, und teils sind es auch aufwendige Bearbeitungsschritte, wie das Schleifen und Polieren der Endfläche, die derzeitige Lichtleiter bzw. Bildleiter vergleichsweise kostenaufwendig machen.

Andererseits müssen aber auch noch gewisse lichttechnische Anforderungen beim endoskopischen Einsatz insbesondere in der Medizintechnik berücksichtigt werden. Dies sind neben möglichst verlustfreier Bereitstellung des von einer Lichtquelle bereitgestellten Lichtes an den Untersuchungsort, farbtreue bzw. gezielt farbige Darstellung des Untersuchungsortes auch die Vermeidung unnötige Wärme an den Untersuchungsort einzubringen. Eine besondere Herausforderung stellt dabei der von der Lichtquelle zur Verfügung gestellte Lichtstrom und die Weiterleitung des Lichtes zum distalen Ende eines Endoskops dar. Insbesondere für Endoskopsysteme mit kleinem Durchmesser bedarf es extrem heller Lichtquellen zum einen und Lichtstrom-optimierte Lichtleiter zum anderen.

Bei Verwendung von aktiven elektronischen Bauelementen, wie beispielsweise Kamera-Chips und/ oder LED zur Beleuchtung müssen zudem noch Anforderungen hinsichtlich elektrischer Isolation, elektrischer Abschirmung, sowie Patientenableitströmen berücksichtigt werden, die je nach Einsatzgebiet des Endoskops maximale Grenzwerte nicht überschreiten dürfen. So ist beispielsweise bei Anwendungen am Herzen ein max. Ableitstrom von 10 µA gefordert, was einer CF-Klassifizierung entspricht (vergl. EN 60601-1, 3. Ausgabe Tab. 3).

Dazu können noch Anforderungen hinsichtlich der Abschirmung insbesondere der Kamera-Chips vor Streulicht aus den Lichtleitern kommen, welche ansonsten die Bildqualität negativ beeinträchtigen kann und/ oder Bildartefakte erzeugen kann.

Neben diesen lichttechnischen und elektrischen Anforderungen sind auch noch Anforderungen hinsichtlich der Biokompatibilität zu beachten. Für die Biokompatibilität ist es erforderlich sicherzustellen, dass das Material mit menschlichem Organismus verträglich ist. Für Medizinprodukte, die in Kontakt zum menschlichen Körper kommen können, ist es regulatorisch gefordert, mögliche Interaktionen und unerwünschte Nebenwirkungen zu bestimmen und zu bewerten. Die Wahl der erforderlichen Prüfungen ergibt sich aus der Kontaktart und der Kontaktdauer im menschlichen Körper. Entsprechend der europäischen Richtlinie für Medizinprodukte MDD 93/42 EWG (kurz MDD) bzw. der Verordnung (EU) 2017/745 vom 5. April 2017 (kurz MDR) ist diese biologische Beurteilung eines Produktes immer dann notwendig, wenn ein unmittelbarer Kontakt von Werkstoff/Produkt mit dem Patienten besteht.

Die Hauptregelwerke zur biologischen Untersuchung und Beurteilung von Werkstoffen sind die DIN EN ISO 10993 und die Prüfung nach United States Pharmacopeia Class VI (USP Class VI). Obwohl die deutlich umfangreichere ISO 10993 ursprünglich den Test nach USP Class VI ersetzen sollte, wird die USP-Prüfung heute insbesondere sehr häufig zur Beurteilung von biokompatiblen Kunststoffen herangezogen. Dazu werden die für eine invasive Applikation vorgesehenen Materialien zum einen hinsichtlich ihrer chemischen Verbindung bewertet und zum anderen einem Zytotoxizitätstest unterzogen, bei dem möglichen toxischen Wirkungen auflebende Zellkulturen untersucht werden. Die Anforderungen hierfür sind in der DIN EN ISO 10993, insbesondere in den Teilen -1 und -5 zusammengefasst (DIN EN ISO 10993-1: 2010-04). In USA unterliegt dies den Anforderungen der FDA. Zur DIN EN ISO 10993 korrespondierende Anforderungen sind dort in der USP Class VI hinterlegt.

Weiterhin kommt der Auslegung der Endoskope als Einweg-Endoskope zu Gute, dass die als Aufbereitungsmethoden bekannten Reinigungs-/ Desinfektionsverfahren mit stark basischen Lösungen und die Sterilisation mittels Autoklavieren bei Temperatur bis zu 135° C und typischen Dampfdrücken von etwa 3 bar bei der Materialauswahl nicht in diesem Maße berücksichtigt werden müssen, was insbesondere auch eine kostengünstigere Materialauswahl erlaubt. Lediglich sind die Eignung für Gas-Sterilisationsverfahren, wie die Ethylenoxid-Sterilisation, sowie RoHS- als auch REACH-Bestimmungen bei den Materialien zu berücksichtigen.

Die eigenen Anmeldungen der Anmelderin mit den Aktenzeichen DE 10 2019 125 912 sowie DE 10 2018 107 523 befassen sich mit verschiedenen Aspekten der Lichtleiter. Eine laserbasierte Lichtquelle wird nicht genannt.

Das US-amerikanische Patent US 6 398 721 B betrifft eine chirurgische Mikroskopievorrichtung, welche eine Laserdiode umfassen kann.

Die US-amerikanische Patentanmeldung US 2006/0279950 A1 beschreibt eine LED. Endoskope werden nicht genannt, allerdings können beispielsweise Lichtleiter umfassend Fasern verwendet werden.

Ebenso beschreibt auch die US-amerikanische Patentanmeldung US 2006/0152926 A1 eine LED, welche beispielsweise auch in Endoskopen verwendet werden kann. Die LED wird in Transmission betrieben.

Eine hocheffiziente Lichtquelle ist in der US-Patentanmeldung US 2004/0246744 A1 beschrieben.

Die US-amerikanische Patentanmeldung US 2019/0290100 A1 beschreibt ein optisches Abbildungssystem, welches insbesondere in der Fluoreszenzmikroskopie (STED-Mikroskopie) Verwendung finden kann.

Die US-amerikanische Patentanmeldung US 2006/0069314 A1 beschreibt eine Festkörperlichtquelle für ein Endoskop.

In der deutschen Patentanmeldung DE 10 2017 108 698 A1 wird ein optoelektronisches Bauelement beschrieben.

Die US-amerikanische Patentanmeldung US 2017/231698 A1 beschreibt ein Endoskop mit einer Beleuchtungs-Lichtfaser.

Im Hinblick auf Endoskopsysteme, insbesondere Single-Use-Endoskope, mit kleinem Durchmesser, z.B. von höchstens 3 mm Gesamtdurchmesser oder weniger, bei denen ein Kamerachip noch mit lichtleitenden Fasern zur Beleuchtung zu montieren ist, hat sich insbesondere der hohe Montageaufwand mit klassischen Faserbündeln mit mehreren Hundert Einzelfasern als nachteilig erwiesen. Zudem steht für Faserbündel dieser Art immer weniger Platz zur Verfügung, so dass auch die Lichtausbeute am distalen Ende eines solchen Endoskops zu einer zusätzlichen Herausforderung wird.

### Aufgabe der Erfindung

Die Aufgabe der Erfindung besteht darin, die Schwächen des Standes der Technik wenigstens teilweise zu überwinden oder zumindest zu mindern, insbesondere aber Endoskopsysteme bereitzustellen, welches insbesondere für den Single-Use-Einsatz eine helle Lichtquelle bzw. eine Beleuchtung mit ausreichend großer Helligkeit und ein dazu optimiertes Lichtleitsystem umfasst, welches sich zudem einfach montieren lässt.

### Zusammenfassung der Erfindung

Die Aufgabe der Erfindung wird gelöst durch den Gegenstand der unabhängigen Ansprüche. Spezielle und bevorzugte Ausführungsformen finden sich in den abhängigen Ansprüchen und der weiteren Offenbarung.

Die Erfindung betrifft daher ein Endoskop mit einem ersten Bauteil und einem zweiten Bauteil, wobei das zweite Bauteil ein mit dem ersten Bauteil verbundenes proximales Ende, vorzugsweise ein lösbar verbundenes proximales Ende, und ein distales Ende aufweist. Im distalen Ende ist ein Element zur Bildaufnahme, wie beispielsweise ein Kamerachip oder ein faseroptisches Element, angeordnet. Im zweiten Bauteil verläuft ein Lichtleiter mit zumindest einer lichtleitenden Faser, um Licht der Lichtquelle vom proximalen Ende zum distalen Ende zu leiten und am distalen Ende abzugeben, sowie vorzugsweise eine Versorgungsleitung zur elektrischen Speisung des Kamerachips.

Im ersten Bauteil kann eine Lichtquelle eingebaut sei, wobei die Lichtquelle mindestens einen LED-Chip zur Abgabe von Licht umfasst und wobei der LED-Chip so mit dem am ersten Bauteil verbundenen proximalen Ende des zweiten Bauteils gekoppelt ist, dass das vom LED-Chip abgegebene Licht in die zumindest eine lichtleitende Faser des Lichtleiter einkoppelbar ist.

Es ist aber auch möglich, dass die Lichtquelle als Komponente einer Steckverbindung in diese Steckverbindung aufgenommen ist. Die Steckverbindung bildet dabei das proximale Ende des zweiten Bauteils mit seinem Lichtleiter. Dabei weist die Lichtquelle mindestens eine in die Lichtquelle eingebaute, vorzugsweise fest eingebaute, LED auf, die ihr abgegebenes Licht in zumindest eine lichtleitende Faser des Lichtleiters einkoppelt.

In jedem Fall weist die mindestens eine lichtleitende Faser einen Durchmesser von mindestens 80 µm auf, wobei die mindestens eine lichtleitende Faser eine zumindest teil- und/oder abschnittsweise an der Mantelfläche angeordnete polymerbasierte Beschichtung und/oder eine Schutzhülle aus einem polymerbasierten Schlauchmaterial aufweist.

Die Beschichtung und/oder die Schutzhülle sind dabei im Regelfall vollflächig an der Mantelfläche angeordnet, also an der gesamten Mantelfläche. Es ist aber vollstellbar, dass die Beschichtung und/oder die Schutzhülle nur teil- oder abschnittsweise an beispielsweise mechanisch besonders beanspruchten Bereichen angeordnet ist. Auch ist es möglich, dass eine Beschichtung und/oder eine Schutzhülle zunächst vollflächig auf die Mantelfläche beschichtet oder auf dieser angeordnet werden, die Beschichtung und/oder die Schutzhülle jedoch nachträglich teil- und/oder abschnittsweise wieder von der mindestens einen lichtleitenden Faser entfernt wird. Dies kann vorteilhaft und/oder notwendig sein an Stellen, an denen die Faser geklebt oder montiert werden soll, beispielsweise am distalen Ende, also beispielsweise in Richtung zu einem Kamerachip.

Faseroptische Elemente zur Bildaufnahme bzw. Bildweiterleitung werden auch als "Image Guides" bezeichnet und bestehen aus einigen Zehntausend Einzelfasern, welche geordnet zueinander an den Endflächen angeordnet sind. Solche faseroptischen Elemente können insbesondere aus Glas oder aus Kunststoff bestehen oder Glas oder Kunststoff umfassen, beispielsweise als Glasfaser bzw. als Kunststofffaser ausgestaltet sein.

Eine solche Ausgestaltung eines Endoskops weist eine Reihe von Vorteilen auf.

Gemäß der vorliegenden Offenbarung ist das Endoskop nämlich in zwei Bauteile aufgeteilt. In das erste Bauteil, welches auch als proximales Bauteil bezeichnet werden kann, kann beispielsweise eine Lichtquelle eingebaut sein, welche mindestens einen LED-Chip zur Abgabe von Licht aufweist, wobei der LED-Chip so mit dem am ersten Bauteil verbundenen proximalen Ende des zweiten Bauteils gekoppelt ist, dass das vom LED-Chip abgegebene Licht in die zumindest eine lichtleitende Faser des Lichtleiters einkoppelbar ist.

In einer Variante des Endoskops kann auch vorgesehen sein, dass die Lichtquelle integraler Bestandteil einer Steckverbindung, also in die Steckverbindung eingebaut, vorzugsweise fest eingebaut, ist, welche das proximale Ende des zweiten Bauteils mit seinem Lichtleiter bildet und wobei die Lichtquelle mindestens eine integrierte, also in die Lichtquelle eingebaute, vorzugsweise fest in die Lichtquelle eingebaute, LED aufweist, die ihr abgegebenes Licht in die zumindest eine lichtleitende Faser des Lichtleiters einkoppelt. Ein derartiger Ansatz wird auch in der Patentschrift DE 102011119972 B4 der Anmelderin beschrieben, wobei hier insbesondere kostengünstige faseroptische Beleuchtungsmöglichkeiten im Vordergrund stehen.

Mit anderen Worten ist das erste Bauteil gemäß beiden vorstehenden Varianten jeweils so ausgestaltet, dass es mit einem zweiten Bauteil, welches auch als distales Bauteil bezeichnet werden kann, verbindbar ausgestaltet ist, oder es liegt sogar mit dem zweiten Bauteil verbunden vor.

Das erste Bauteil kann je nach genauer Ausgestaltung und je nach der Art der von ihm umfassten Elemente beispielsweise als Handstück ausgestaltet sein, also beispielsweise als ein Bauteil, welches auch dazu dient, das Endoskop zu handhaben und/oder zu halten. Es ist aber auch möglich, dass das erste Bauteil Elemente umfasst, welche zur Steuerung und/oder zum Betrieb eines Endoskops dienen, beispielsweise also als Steuer- und/oder Auswerteeinheit ausgestaltet sind, so dass in diesem Fall das erste Bauteil auch als ein Betriebsgerät für das Endoskop ausgestaltet sein kann.

Weiterhin umfasst das Endoskop ein zweites Bauteil, welches ein proximales und ein distales Ende aufweist und wobei im zweiten Bauteil ein Lichtleiter, der zumindest eine lichtleitende Faser umfasst, verläuft. Der Lichtleiter ist ausgestaltet, um Licht der Lichtquelle vom proximalen Ende zum distalen Ende zu leiten und am distalen Ende abzugeben. Am distalen Ende ist ein Element zur Bildaufnahme, wie ein Kamerachip zur Bildaufnahme oder ein faseroptischer Bildleiter, angeordnet. Weiterhin umfasst das zweite Bauteil vorzugsweise für den Fall, dass das distale Ende einen Kamerachip umfasst, eine Versorgungsleitung zur elektrischen Speisung des Kamerachips.

Eine solche Ausgestaltung des Endoskops mit zwei Bauteilen (oder auch Baugruppen) ist vorteilhaft. Denn das Endoskop ist gemäß der beschriebenen Ausgestaltung so aufgebaut, dass das erste Bauteil Elemente umfasst, wie beispielsweise die Lichtquelle, welche relativ kostenintensiv sind, und das zweite Bauteil hingegen solche Elemente umfasst, die relativ kostengünstig sind. Es ist also möglich, das Endoskop aufzutrennen, und auf diese Weise können beispielsweise preiswerte Elemente in einer vergleichsweise preiswerten Single-Use-Baugruppe untergebracht werden, wohingegen die weniger preiswerten, kostenintensiven Elemente in einer Multi-Use-Baugruppe untergebracht sind.

Damit es nun erstmals möglich, beispielsweise ein Endoskop bereitzustellen, dass die Vorteile einer sehr hochwertigen Beleuchtung mit den Vorteilen eines lediglich für den einmaligen Gebrauch vorgesehenen Endoskops zu verbinden. Beachtlich ist hierbei, dass das Endoskop nach der vorliegenden Offenbarung aber nicht zwingend als Einmal-Endoskop bzw. als zumindest teilweise als Einmal-Endoskop ausgestaltet sein muss. Vielmehr ist es auch denkbar, dies bedarfsweise anzupassen.

Es kann jedoch von Vorteil sein, wenn erste Bauteil und zweites Bauteil so miteinander verbunden sind, dass sie voneinander lösbar sind. Sofern das Endoskop als zumindest teilweise für den Einzelgebrauch vorgesehenes Endoskop ausgebildet ist, kann beispielsweise das zweite Bauteil nach Gebrauch entsorgt werden. Es ist aber auch möglich, dass das zweite Bauteil zwar lösbar mit dem ersten Bauteil verbunden ist, aber dennoch für den mehrfachen Gebrauch vorgesehen ist, und nach der Trennung vom ersten Bauteil bestimmten, für den medizinischen Gebrauch vorgesehenen Reinigungs- und Sterilisationsprozessen unterzogen wird.

Mit dem Endoskop nach der vorliegenden Offenbarung, welches auch als modulares Endoskop beschrieben werden kann, ist also zum einen auch die Möglichkeit einer vereinfachten Handhabung verbunden. Zum anderen kann, gerade wenn es sich um ein Einweg-Endoskop oder ein zumindest teilweise als Einweg-Endoskop ausgebildetes Endoskop handelt, also um ein Endoskop, von dem zumindest einige Bestandteile nur für den einmaligen Gebrauch bestimmt sind, eine hochwertige Beleuchtung, wie beispielsweise die Beleuchtung mittels eines Lasers, die eine hohe Lichtintensität ermöglicht, oder aber auch mit einer Hochleistungs-LED als Lichtquelle mit den Vorzügen eines Einweg-Geräts verbunden werden.

Das zweite Bauteil, welches einen Lichtleiter umfasst, kann beispielsweise starr ausgeführt sein, oder auch flexibel. Allgemein kann das zweite Bauteil auch als sogenannter Schaft eines Endoskops verstanden werden, wobei im Rahmen der vorliegenden Offenbarung unter einem Schaft allgemein sowohl ein starres zweites Bauteil verstanden wird als auch ein flexibles Bauteil, welches beispielsweise nur einen flexiblen äußeren Hüllschlauch beispielsweise umfassend ein Kunststoffmaterial umfasst. Sofern das zweite Bauteil starr ausgeführt ist, kann es beispielsweise so ausgestaltet sein, dass der Lichtleiter, welcher vom zweiten Bauteil umfasst wird, zumindest abschnittsweise von einem Rohrabschnitt oder von mehreren Rohrabschnitten umfassend ein Metall oder einen Kunststoff umgeben ist. Die genaue Ausführung des zweiten Bauteils kann dabei je nach bevorzugtem Einsatzgebiet des Endoskops ausgewählt sein.

Geeignete Lichtleiter können beispielsweise einige Zehn, einige Hundert bis hin zu einigen Tausend Einzelfasern für derartige Endoskopsysteme umfassen, wobei die genaue Zahl der Einzelfasern, welche vom Lichtleiter umfasst sind, beispielsweise abhängig ist vom adressierten Enddurchmesser des Lichtleiters und/oder dem Durchmesser vom Lichtleiter umfassten Einzelfasern. Die normal üblichen Faser-Durchmesser betragen zwischen 20 µm und 100 µm. Typisch sind Durchmesser von 30 µm, 50 µm und 70 µm.

Insbesondere für Single-Use-Endoskope bzw. für Endoskopsysteme mit kleinen Abmessungen kann vorteilhaft sein, wenige dicke Fasern als lichtleitende Fasern zu verwenden, um sicherzustellen, dass eine ausreichende Leuchtdichte bzw. Beleuchtungsintensität am zu untersuchenden Bereich gewährleistet ist. Einerseits kann damit eine kostengünstige schnelle Montage erfolgen und anderseits ein hoher Lichtstrom von der Laser-Lichtquelle kommend zum distalen Ende des Endoskops gewährleistet werden.

Als vorteilhaft und als guter Kompromiss zwischen Montageaufwand und ausreichender Lichtstromübertragung hat sich eine Anzahl von höchstens zwanzig derartiger Einzelfasern herausgestellt, bevorzugt höchstens zehn derartiger Einzelfasern, wobei für extrem dünne Endoskopsysteme bereits eine Faser ausreichend sein kann. Bündel aus drei oder sieben Einzelfasern bieten den Vorteil, dass diese sehr dicht in einer gemeinsamen Hülse gepackt werden können. Die 7er-Anordnung bildet dabei den besonderen Vorteil, dass sich in der gemeinsamen Hülse eine eher kreisrunde Anordnung der Einzelfasern verwirklichen lässt und zudem eine für Fasern mit rundem Querschnitt ideale Packungsdichte ergibt. Bei einer solchen 7er-Anordnung können dann beispielsweise am distalen Ende des Endoskops die Einzelfasern um den Kamerachip oder um die Bildleiter derart gruppiert werden, dass eine gleichmäßige Ausleuchtung des zu untersuchenden Gewebes ermöglicht werden kann. Im Hinblick auf die eher übliche quadratische Chip-Form von Laser- oder Leuchtdioden oder des Konverters als Lichtquelle kann es aber auch vorteilhaft sein, wenn vier Enzelfasern oder ganzzahlige Vielfache von vier derartiger Fasern verwendet werden. Einerseits können die für die Beleuchtung zur Verfügung stehenden Kavitäten hinsichtlich einer möglichst hohen aktiven Faserfläche, also der eigentlich lichtleitende Querschnittsbereich der Faser, mit mehr Fasern gefüllt werden, und anderseits eine bessere Lichteinkopplung erzielt werden.

Hierbei hat sich als vorteilhaft erwiesen, dass die eine lichtleitende Faser oder die mehreren lichtleitenden Fasern einen Durchmesser im Bereich von 100 µm bis 1000 µm, bevorzugt bis 500 µm, vorzugsweise im Bereich von 150 µm bis 400 µm, aufweisen. Derartige Fasern lassen sich als Einzelfaser deutlich einfacher montieren und weisen noch einen ausreichend kleinen minimalen Biegeradius auf. Für heutige Endoskope mit beispielsweise einem Kamera-Chip der Grüße 1 x 1 mm² wären beispielsweise vier Enzelfasern, eine an jeder Seite der Kamera angeordnet, mit einem Durchmesser im Bereich von 200 µm bis 300 µm ideal. Ebenso bevorzugt sind Anordnungen mit in Summe acht oder zwölf Einzelfasern, je zwei oder drei Fasern an jeder Seite der Kamera angeordnet, wobei in diesem Fall die Einzelfasern einen Durchmesser im Bereich von 150 µm bis höchstens 200 µm aufweisen. Es kann auch vorgesehen sein, dass beispielsweise Fasern mit unterschiedlichem Durchmesser verwendet werden, um eine möglichst gute Ausfüllung der Kavität zwischen Kamera-Chip und umgebender Hülle zu ermöglichen, so dass ein möglichst hoher Lichtstrom erzielt werden kann. Bei einer 12er-Faseranordnung, drei Fasern pro Kamera-Seite, könnte beispielsweise die mittlere Faser einen Durchmesser von etwa 250 µm aufweisen, wobei die beiden anderen Fasern nur einen Durchmesser von 100 µm bis 150 µm aufweisen.

Grundsätzlich können statt der Einzelfasern auch dünne Faserbündel Verwendung finden, welche insbesondere aus sehr dünnen Einzelfasern mit einem Einzelfaserdurchmesser von bevorzugt kleiner 70 µm, besonders bevorzugt kleiner 50 µm bestehen, welche nur einen extrem dünnen Mantel aufweisen, welcher das Faserbündel zusammenhält. Derartige Faserbündelausführungen sind in einer noch nicht veröffentlichten Parallel-Anmeldung der Anmelderin beschrieben.

Gemäß einer weiteren Ausführungsform ist die eine lichtleitende Faser oder sind die mehreren lichtleitenden Fasern Stufenindex-Glasfasern. Vorzugsweise ist die eine lichtleitende Faser oder sind die mehreren lichtleitenden Fasern Stufenindex-Glasfasern umfassend eine Glaszusammensetzung, die bis auf unvermeidliche Spuren frei ist von Blei und/oder sonstigen Schwermetallen sowie frei von Antimon und/oder Arsen und/oder von sonstigen kritischen Elementen wie Cr(VI) ist.

Unter einer Faser wird im Rahmen der vorliegenden Offenbarung ein Körper verstanden, dessen größte laterale Abmessung in einer Raumrichtung eines kartesischen Koordinatensystems mindestens um den Faktor 10, vorzugsweise mindestens um den Faktor 50, größer ist als in den beiden anderen, zu dieser ersten Raumrichtung senkrechten Raumrichtungen. Mit anderen Worten ist eine Faser ein sehr langer, dünner Körper.

Unter einer Stufenindex-Glasfaser wird im Rahmen der vorliegenden Offenbarung eine Glasfaser verstanden, deren Brechungsindex sich von innen, dem Kern, nach außen, in Form mindestens einer Stufe ändert. Dabei umfasst die Glasfaser ein Kernglas und ein Mantelglas, wobei das Kernglas einen anderen Brechungsindex aufweist als das Mantelglas.

Eine Glasfaser umfasst Glas. Die Glasfaser kann weiterhin neben dem glasigen Material noch ein weiteres Material umfassen, welches die Oberfläche des glasigen Materials zumindest teilweise umgibt, die sogenannte Schlichte. Je nach Einsatzzweck können unterschiedliche glasige Materialien für eine Glasfaser verwendet werden. Insbesondere kann die Glasfaser ein einkomponentiges und/oder ein mehrkomponentiges Glas umfassen. Beispielsweise kann die Glasfaser als im Wesentlichen einkomponentiges Glas Quarzglas umfassen und/oder insbesondere als Quarzglasfaser ausgebildet sein, wobei das Quarzglas auch dotiert vorliegen kann, beispielsweise mit OH-Ionen und/oder mit Fluor dotiert, und/oder als bspw. wasserreiche oder wasserarme Quarzglasvarianten vorliegt, wobei in diesem Fall noch immer von einem einkomponentigen Glas gesprochen wird, oder ein mehrkomponentiges Glas umfassen, beispielsweise ein mehrkomponentiges, silikatisches Glas. Weiterhin kann das Glas auch als Chalkogenidglas ausgebildet sein. Unter einer Quarzglasfaser oder Quarzfaser wird dabei auch eine Faser umfassend dotiertes Quarzglas verstanden.

Bevorzugt umfasst die optische Faser einen Faserkern und einen Faserrand bzw. eine Fasermantelschicht. In bevorzugten Ausführungsformen besteht der Kern, bzw. die Kernschicht, aus einem Kernglas.

Bevorzugt umfasst die optische Faser einen Fasermantel, welche den Faserkern umgibt. In bevorzugten Ausführungsformen umfasst der Fasermantel ein Mantelglas.

Bevorzugt weist der Fasermantel einen Gehalt an Halogenen bzw. Halogeniden von weniger als 500 ppm (m/m) auf, weiter bevorzugt von weniger als 400 ppm (m/m), weiter bevorzugt von weniger als 300 ppm (m/m), weiter bevorzugt von weniger als 250 ppm (m/m), weiter bevorzugt von weniger als 200 ppm (m/m), weiter bevorzugt von weniger als 150 ppm (m/m), weiter bevorzugt von weniger als 100 ppm (m/m), weiter bevorzugt von weniger als 80 ppm (m/m), weiter bevorzugt von weniger als 60 ppm (m/m), weiter bevorzugt von weniger als 40 ppm (m/m), weiter bevorzugt von weniger als 20 ppm (m/m), noch weiter bevorzugt von weniger als 10 ppm (m/m). In besonders bevorzugten Ausführungsformen ist der Fasermantel frei von Halogen. Halogene sind beispielsweise Chlor, Fluor, Brom und/oder lod bzw. deren Anionen. Eine zu hohe Konzentration an Halogenen Im Fasermantel führt zur Bildung der entsprechenden Halogensäuren, insbesondere beispielsweise bei der Dampfsterilisation. Die entsprechenden Halogensäuren können die Beständigkeit des optischen Faserartikels mindern sowie aus diesem austreten. Insbesondere greifen die Halogensäuren Materialien wie beispielsweise Edelstahl von Autoklaven und Endoskopen an und führen zur Bildung von unerwünschtem Rost.

Bevorzugt weist der Faserkern einen Gehalt an Halogenen bzw. Halogeniden von weniger als 500 ppm (m/m) auf, weiter bevorzugt von weniger als 400 ppm (m/m), weiter bevorzugt von weniger als 300 ppm (m/m), weiter bevorzugt von weniger als 250 ppm (m/m), weiter bevorzugt von weniger als 200 ppm (m/m), weiter bevorzugt von weniger als 150 ppm (m/m), weiter bevorzugt von weniger als 100 ppm (m/m), weiter bevorzugt von weniger als 80 ppm (m/m), weiter bevorzugt von weniger als 60 ppm (m/m), weiter bevorzugt von weniger als 40 ppm (m/m), weiter bevorzugt von weniger als 20 ppm (m/m), noch weiter bevorzugt von weniger als 10 ppm (m/m). In besonders bevorzugten Ausführungsformen ist die Kernschicht bzw. der Kern frei von Halogen. Erfindungsgemäße Halogene sind beispielsweise Chlor, Fluor, Brom und/oder lod bzw. deren Anionen. Eine zu hohe Konzentration an Halogenen im Faserkern führt zur Bildung der entsprechenden Halogensäuren, insbesondere beispielsweise bei der Dampfsterilisation. Die entsprechenden Halogensäuren können die Beständigkeit des optischen Faserartikels mindern sowie aus diesem austreten. Insbesondere greifen die Halogensäuren Materialien wie beispielsweise Edelstahl von Autoklaven und Endoskopen an und führen zur Bildung von unerwünschtem Rost.

In bestimmten Ausführungsformen ist die optische Faser eine Quarzfaser. In einer bestimmten Ausführungsform weist der Fasermantel und/oder weist der Faserkern einen Anteil von mindestens 76 Gew.-% SiO₂, beispielsweise in amorpher Form, auf, weiter bevorzugt mindestens 81 Gew.-%, weiter bevorzugt mindestens 84 Gew.-%, weiter bevorzugt mindestens 88 Gew.-%, weiter bevorzugt mindestens 92 Gew.-%, weiter bevorzugt mindestens 95 Gew.-%, weiter bevorzugt mindestens 97 Gew.-%, weiter bevorzugt mindestens 98 Gew.-%. Ein höherer Quarzanteil führt zu einer erhöhten chemischen Beständigkeit sowie zu einer erhöhten Temperaturbeständigkeit. Unter einer Quarzfaser wird also insbesondere eine Quarzglasfaser verstanden, d.h. aus oder umfassend amorphes SiO₂.

In einer bestimmten Ausführungsform weist das Kernglas folgende Merkmale auf:
Bevorzugt umfasst das Kernglas wenigstens 8 Gew.-%, weiter bevorzugt wenigstens 23 Gew.-%, mehr bevorzugt wenigstens 24 Gew.-% und besonders bevorzugt wenigstens 25 Gew.-% oder sogar wenigstens 26 Gew.-% SiO₂. In einer besonderen Ausführungsform kann das Kernglas sogar mindestens 28,3 Gew.-% SiO₂ umfassen, ganz besonders bevorzugt mindestens 34 Gew.-% SiO₂. In einigen bevorzugten Ausführungsformen umfasst das Kernglas sogar wenigstens 35 Gew.-% SiO₂, weiter bevorzugt wenigstens 42 Gew.-%.

Bevorzugte Kerngläser dieser Erfindungen umfassen die folgenden Komponenten in den folgenden Zusammensetzungsbereichen in Gewichtsprozent:

| **Komponente** | **von** | **bis** |
|---|---|---|
| B₂O₃ | 0 | 24 |
| SiO₂ | 23 | 62,1 |
| Al₂O₃ | 0 | 10 |
| Li₂O | 0 | 10 |
| Na₂O | 0 | 18,5 |
| K₂O | 0 | 25,7 |
| BaO | 0 | 57,8 |
| ZnO | 0 | 40 |
| La₂O₃ | 0 | 25 |
| ZrO₂ | 0 | 10 |
| HfO₂ | 0 | 14,2 |
| SnO₂ | >0 | 2 |
| MgO | 0 | 8 |
| CaO | 0 | 8 |
| SrO | 0 | 24,4 |
| Ta₂O₅ | 0 | 22 |
| Y₂O₃ | 0 | 11,9 |
| Rb₂O | 0 | 15 |
| Cs₂O | 0 | 21 |
| GeO₂ | 0 | 7,5 |
| F | 0 | 2 |
| Σ R₂O | 5 | 20 |
| Σ MgO, CaO, SrO, ZnO | 20 | 42 |

| | | |
|---|---|---|
| R₂O ist jeweils die Summe der Gehalte aller Alkalimetalloxide. | | |

Eine oder mehrere der folgenden Komponenten kann vom Kernglas umfasst sein: Cs₂O, Rb₂O, MgO, CaO, SrO, Gd₂O₃, Lu₂O₃, Sc₂O₃, Y₂O₃, In₂O₃, Ga₂O₃ und WO₃.

Folgende Komponenten sollten im Kernglas bevorzugt nicht enthalten sein oder lediglich in Konzentrationen von jeweils höchstens 500 ppm, die durch unvermeidbare Verunreinigungen der Rohstoffe bedingt sind: TiO₂, CeO₂, Nb₂O₅, MoOs, Bi₂O₃, PbO, CdO, TI₂O, As₂O₃, Sb₂O₃, SO₃, SeO₂, TeO₂, BeO, radioaktive Elemente und färbende Komponenten, soweit im Text nicht anders beschrieben. Insbesondere auf TiO₂ sollte verzichtet werden, weil diese Komponente für eine ausgeprägte Absorption im UV-Bereich führen kann. In bevorzugten Ausführungsformen wird auch auf die Komponente WO₃ verzichtet.

Die Komponenten TiO₂, CeO₂, Nb₂O₅ und/oder Bi₂O₃ können bis maximal 0,5 Gew.-%, bevorzugt bis 0,3 Gew.-% und besonders bevorzugt bis 0,2 Gew.-% im Kernglas enthalten sein. In einer bevorzugte Ausführungsform ist das Kernglas frei von diesen Komponenten.

Bevorzugt ist das Kernglas frei von optisch aktiven Komponenten, insbesondere Sm₂O₃, Nd₂O₃, Dy₂O₃, Pr₂O₃, Eu₂O₃, Yb₂O₃, Tb₂O₃, Er2Ü3, Tm₂O₃ und/oder Ho₂O₃. CeO₂ absorbiert im UV-Bereich, so dass bevorzugte Kerngläser kein CeO₂ enthalten.

Der Gehalt der Komponenten Erdalkalimetalloxide, La₂O₃, Ta₂O₅, ZrO₂ und HfO₂ beträgt in Summe vorzugsweise und insbesondere für Kerngläser mit Brechwerten von mehr als 1,65 wenigstens 40 Gew.-%, weiter bevorzugt wenigstens 42 Gew.-%, mehr bevorzugt wenigstens 50 Gew.-% und besonders bevorzugt wenigstens 55 Gew.-%. Wenn der Gehalt dieser Komponenten zu niedrig ist, kann der bevorzugte Brechungsindex normalerweise nicht erreicht werden. Formulierungsbedingt sollte diese Summe einen Wert von 72 Gew.-% nicht übersteigen.

In einer bestimmten Ausführungsform weist das Mantelglas folgende Merkmale auf:
Bevorzugt weist das Mantelglas einen Gehalt an SiO₂ von >60 Gew.-%, weiter bevorzugt >65 Gew.-% und besonders bevorzugt von mindestens 69 Gew.-% auf. Der SiO₂-Gehalt beträgt bevorzugt höchstens 75 Gew.-% und besonders bevorzugt bis zu 73 Gew.-%. Das Mantelglas ist tendenziell stärkeren Umwelteinflüssen ausgesetzt als das Kernglas. Ein hoher SiO₂-Gehalt verleiht bessere chemische Resistenz. Folglich ist der Gehalt an dieser Komponente im Mantelglas bevorzugt größer als im Kernglas.

Vorzugsweise wird die Zusammensetzung des Mantelglases so ausgewählt bzw. an die des Kernglases angepasst, dass der lineare thermische Ausdehnungskoeffizient des Mantelglases und derjenige des Kernglases sich möglichst wenig unterscheiden. Allgemein kann der thermische Ausdehnungskoeffizient (CTE) in einem Temperaturbereich von 20 bis 300°C für Faserkern und Fasermantel gleich oder unterschiedlich sein. Insbesondere ist der CTE unterschiedlich. Vorzugsweise ist der CTE des Mantels kleiner als der CTE des Faserkerns, typischerweise ist er mindestens um 1,0*10⁻⁶ /K kleiner, kann aber auch, je nach Glas typischerweise mindestens um 2,5*10⁻⁶ /K. kleiner sein. Der Faserkern weist typischerweise einen CTE von 6,5*10⁻⁶ bis 10*10⁻⁶/K auf, der Mantel einen CTE von 4,5*10⁻⁶ bis 6*10⁻⁶/K. Dadurch wird erreicht, dass der Kern der Faser beim Abkühlen stärker als der Fasermantel schrumpft, wodurch im Fasermantel eine die Faser schützende Druckspannung aufgebaut wird, was für die mechanische Belastbarkeit der Faser, insbesondere ihrer Biegefestigkeit zuträglich ist.

Die folgende Tabelle zeigt einige bevorzugte Zusammensetzungen von Mantelgläsern, die zusammen mit den Kerngläsern verwendet werden können. Die Mantelgläser umfassen (in Gew.-% auf Oxidbasis):

| **Oxide** | **Gruppe 1** | **Gruppe 2** | **Gruppe 3** | **Gruppe 4** |
|---|---|---|---|---|
| SiO₂ | 70 - 78 | 63 - 75 | 75 - 85 | 62 - 70 |
| Al₂O₃ | 5-10 | 1 - 7 | 1-5 | 1-10 |
| B₂O₃ | 5-14 | 0-3 | 10-14 | >15 |
| Li₂O | frei | 0-1 | 0-3 | < 0,1 |
| Na₂O | 0-10 | 8-20 | 2-8 | 0-10 |
| K₂O | 0-10 | 0-6 | 0-1 | 0-10 |
| MgO | 0-1 | 0-5 | frei | 0-5 |
| CaO | 0-2 | 1-9 | frei | 0-5 |
| SrO | 0-1 | frei | frei | 0-5 |
| BaO | 0-1 | 0-5 | frei | 0-5 |
| Halogen | frei | frei | frei | frei |

In einer anderen bestimmten Ausführungsform ist das Kernglas und/oder das Mantelglas ein Chalkogenidglas, welches insbesondere Anwendungen im Infrarot-Bereich ermöglicht. Die folgende Tabelle zeigt bevorzugte Zusammensetzungen von Kernchalkogenidgläsern und/oder Mantelchalkogenidgläsern in Molprozent:

| **Komponente** | **Mol-%** |
|---|---|
| S | 50-90 |
| Ga | 0-25 |
| As | 0-40 |
| Ge | 0-35 |
| R¹ (zugegeben in Form von R¹Hal) | 0-7,25 |
| R² (zugegeben in Form von R²Hal) | 0-13,5 |
| M¹ (zugegeben in Form von M¹Hal₂) | 0-5 |
| M² (zugegeben in Form von M²Hal₂) | 0-7,25 |
| Ln (zugegeben in Form von LnHals) | 0-4 |
| Sum of Ga, As, and Ge | 10-42 |
| Sum of R¹, R², M¹, M², and Ln | 0-16 |
| Sum of Hal | 0-16 |

Hierbei ist Hal = Fluor, Chlor, Brom, und/oder lod; Hal₂ und/oder Hals = Chlor und/oder Brom; R¹ = Li, Na, K, Rb, und/oder Cs; R² = Ag und/oder Cu; M¹ = Mg, Ca, Sr, und/oder Ba; M² = Zn, Cd, Hg, und/oder Pb; Ln = La, Ce, Pr, Nd, Pm, Sm Eu, Gd, Tb, Dy, Ho, Er, Tm, Ty, Lu, Y, und Sc.

Besonders vorteilhaft ist es, wenn die Glasfasern, Faserstäbe oder gepressten Faserstäbe aus einem Pb- bzw. Schwermetall-freien Kernglas und Mantelglas bestehen. Derartige Fasersysteme bieten insbesondere eine hohe Transmission im VIS-Spektralbereich und zeigen aufgrund der vergleichsweisen hohen Transmission im blauen Spektralbereich eine hohe Farbtreue, was insbesondere wichtig ist bei der medizinischen Beurteilung von Gewebe. Oft entscheiden hier nur geringfügige Farbunterschiede des Gewebes, ob es sich um eine gutartige oder bösartige Gewebeveränderung handelt. Daher kommt es auf einen hohen CRI-Wert des Gesamtsystems aus Lichtquelle, Lichtleiter und bildgebender Einrichtung an, wobei CRI (Color Rendering Index) eine Kennzahl einer photometrischen Größe ist, mit der die Qualität der Farbwiedergabe von Lichtquellen gleicher korrelierter Farbtemperatur beschrieben wird. Ein CRI-Wert von > 90 kann mit den oben beschriebenen Glasfasern, Faserstäbe oder gepressten Faserstäbe erzielt werden. Derartige Fasersysteme sind seitens der Anmelderin unter dem Namen SCHOTT PURAVIS^{®} bekannt und sind hinsichtlich ihrer Zusammensetzungen in der DE 102012100233 B4 und DE 102013208838 B4 beschrieben. Ähnliche Fasersysteme sind auch in der EP 2072477 B1 beschrieben, welche ebenfalls Pb-frei sind.

Insbesondere für die Verwendung in Endoskopen ist es von Vorteil, wenn Glasfasern, Faserstäbe oder gepressten Faserstäbe aus einem Glassystem bestehen, welches für das zu leitende Licht einen Akzeptanzwinkel 2α von größer 80°, besonders bevorzugt von größer 100° aufweist, was einer numerischen Apertur (NA) von größer 0,64, besonders bevorzugt größer 0,77 entspricht. Zum einen kann erreicht werden, dass insbesondere Licht von LEDs, welche i.d.R. einen sehr breiten Abstrahlwinkel aufweisen, ohne komplexe Optiken am proximalen Ende in die Glasfasern bzw. Faserstäbe oder gepressten Faserstäbe einkoppelbar sind, ohne dass erhöhte Einkoppelverluste entstehen. Andererseits kann am distalen Ende eine weitwinklige Ausleuchtung ohne zusätzlich erforderliche Optik erreicht werden, was insbesondere für endoskopische Untersuchungen bevorzugt ist. Eine optimale Ausleuchtung bei den derzeit üblichen Kamera-Sichtwinkeln (üblicherweise 120° diagonal) kann dann erzielt werden, wenn die Glasfasern, Faserstäbe oder gepressten Faserstäbe einen Akzeptanzwinkel 2α von mindestens 120° bzw. eine NA von mindestens 0,86 aufweisen.

Glasfasern, wie sie zuvor beschrieben worden, weisen nach Ihrem Ziehprozess normalerweise eine weitgehend unverletzte, feuerpolierte Oberfläche auf, die es gilt, möglichst vor Verletzungen zu schützen. Dazu werden bei Glasfasern sogenannte Schlichten vor dem Wickel-Prozess aufgebracht, die die Fasern insbesondere beim gegenseitigen Reiben der Fasern untereinander aber auch bei Kontakt mit z.B. Metalloberflächen schützen. Derartige Schlichten bestehen i.d.R. aus Wachs- bzw. Sterin-basierten Lösungen, die auf die Glasfasern aufgesprüht werden. In einer noch nicht veröffentlichten Parallelanmeldung der Anmelderin werden weitere dieser Schlichten beschrieben.

Hinsichtlich einer weiteren mechanischen Stabilisierung der Faser, insbesondere bei Fasern mit größerem Durchmesser, wie zuvor beschrieben, hat sich als vorteilhaft herausgestellt, wenn die eine oder die mehreren lichtleitenden Fasern eine zumindest teil- und/oder abschnittsweise an deren Mantelfläche angeordnete polymerbasierte Beschichtung oder eine Schutzhülle aus einem polymerbasiertem Schlauchmaterial, z.B. als Schrumpfschlauch ausgeführt, aufweisen. Dabei kann eine höhere Festigkeit und damit auch kleine Biegeradien der Fasern erzielt werden. Der an sich resultierende Nachteil einer dickeren Faser hinsichtlich zunehmender Steifigkeit und Zunahme des minimal zulässigen Biegeradius kann mit dieser Maßnahme deutlich abgeschwächt oder kompensiert werden.

Gemäß einer Ausführungsform umfasst der Lichtleiter mehrere lichtleitende Fasern, wobei wenigstens eine lichtleitende Faser, bevorzugt mehrere lichtleitende Fasern, besonders bevorzugt alle lichtleitenden Fasern, eine zumindest teil- und/oder abschnittsweise an deren Mantelfläche angeordnete polymerbasierte Beschichtung und/oder eine Schutzhülle aus einem polymerbasiertem Schlauchmaterial aufweist.

Vorteilhaft sind Beschichtungen umfassend ein oder mehrere oder aus einer oder mehreren Acrylat-, Polyamid-, Polyurethan-, Polyimid-, Epoxi-, Ethylen-Tetrafluorethylen-Copolymer- oder Poly-Xylol-basierten Verbindungen (auch als Poly-Xylylen-basierte Beschichtungen bezeichnet), beispielsweise basierend auf poly-para-Xylol-Verbindungen, beispielsweise auch unter dem Handelsnamen "Parylene" als Beschichtungsmaterial bekannt, oder Mischungen dieser Verbindungen. Geeignete Beschichtungsmaterialien sind beispielsweise unter den Handelsnamen oder Marken oder Bezeichnungen NYLON^{®} (Polyamid) oder TEFZEL^{®} oder Parylene^{®} oder PMMA (Polymethylmethacrylat) als Coatings oder Beschichtungen oder Beschichtungsmaterialien erhältlich. Üblicherweise erfolgt bei diesen Schichten eine Aushärtung durch Aufheizen oder mittels UV-Licht. Alternativ oder zusätzlich kann die Beschichtung auch umfassen thermoplastische Elastomere, beispielsweise ein thermoplastisches Polyesterelastomer oder ein thermoplastisches Copolyesterelastomer, wie es beispielsweise unter der Marke HYTREL kommerziell erhältlich ist, oder ein Silikon.

In speziellen Fällen können auch metallische Beschichtungen, beispielsweise aus Gold oder Aluminium zum Einsatz kommen. Derartige leitfähige Beschichtungen können dann z.B. von Vorteil sein, wenn es gilt zudem elektrische Felder abzuschirmen und/ oder elektrische Potentiale zu vermeiden, was beispielsweise bei kardiologischen Anwendungen am Herzen, bei denen eine CF-Klassifizierung erforderlich ist.

Besonders vorteilhaft ist es, wenn derartige Beschichtungen mittels Tauchen, Besprühen, Extrudieren oder Abscheidung bei niedrigem Druck auf die eine oder die mehreren lichtleitenden Fasern unmittelbar nach dem Ziehen der Fasern aufgebracht werden. Damit kann erreicht werden, dass die nahezu perfekte, feuerpolierte Oberfläche der Faser konserviert werden kann, bevor sie in Kontakt mit anderen Materialien oder mit anderen Fasern kommt, und bevor dabei Mikro-Beschädigungen auftreten können, die die Festigkeit der Faser reduzieren. Zudem kann ein Schutz vor hydrolytischem Angriff erzielt werden.

Üblicherweise werden derartige Schichten derart aufgebracht, dass der frisch gezogene Lichtleiter als Faser durch einen Topf mit einer Düse gezogen wird, in dem sich das zu Beschichtung dienende Polymermaterial befindet, wobei mit der Düse u.a. auch die Schichtdicke eingestellt werden kann.

Es ist beispielsweise möglich (siehe beispielhaft die Zusammenfassung zum Kapitel "Fiber coating" von D. Hewak in "Encyclopedia of Modern Optics"), dass das Polymermaterial mittels UV-Licht aushärtbar ausgestaltet ist, oder dass die Beschichtung sich in eine innere und eine äußere Beschichtung aufteilt, wobei die innere Beschichtung weicher (mit kleinerem E-Modul) und die äußere Beschichtung härter (mit höherem E-Modul) ausgebildet sein kann, was insbesondere vorteilhaft sein kann, um Spannungen beim Biegen solcherart beschichteter Fasern zu minimieren. Aber auch andere Beschichtungen, umfassend Metall, Keramik oder Kohlenstoff als Beschichtungsmaterial, sind nach Hewak prinzipiell möglich.

Zudem kann ergänzend vorgesehen sein, dass zusätzlich zu dieser ersten Beschichtung noch mindestens eine weitere organische Beschichtung vorgenommen werden kann. Derartige zusätzliche Beschichtungen werden auch als Buffer bezeichnet und finden üblicherweise bei Quarzfasern Anwendung. Derartige zusätzliche Beschichtungen werden auch als Buffer bezeichnet und finden üblicherweise bei Quarzfasern Anwendung. Als Materialien für derartige Buffer kommen beispielsweise PMMA, Polyamid (NYLON), Polyimid oder ein oder mehrere fluorierte Polymere, wie ein Ethylen-Tetrafluorethylen-Copolymer (Kurzzeichen ETFE), welches beispielsweise unter dem Handelsnamen TEFZEL^{®} kommerziell erhältlich ist, in Betracht. Eine derartige weitere Beschichtung dient zur Steigerung der Robustheit hinsichtlich der Biegebelastbarkeit. Insbesondere kann diese Bufferschicht auch umfassen ein thermoplastisches Elastomer, beispielsweise ein thermoplastisches Polyesterelastomer oder ein thermoplastisches Copolyesterelastomer, wie es beispielsweise unter dem Handelsnamen Hytrel^{®} kommerziell erhältlich ist, und oder Polynivylidenfluorid, beispielsweise erhältlich unter dem Handelsnamen Kynar^{®}, oder Polytetrafluoroethylen (beispielsweise erhältlich unter dem Handelsnamen Teflon^{®}) oder auch Polyurethan. Solche Buffer-Beschichtungen können beispielsweise aufgebracht werden durch Sprühen, Tauchen, Extrusion und elektrostatische Methoden.

Ein derartiges Schichtsystem kann beispielsweise aus einem 2-lagigen System bestehen, bei dem zunächst eine vergleichsweise dünne Schicht, typischerweise 10 µm bis 50 µm dick, z.B. aus einer Acrylat- oder Epoxi-Verbindung, auf den Lichtleiter appliziert wird, und anschließend nochmals als weiterer mechanischer Schutz eine sogenannte Buffer-Schicht aus NYLON, TEFZEL^{®}, PMMA oder Polyimid aufgebracht wird, die dann auch eine deutlich größere Wandstärke, typ. 50 µm bis 200 µm aufweisen kann.

Die Schichtdicke dieser Beschichtung liegt typischerweise im Bereich von 5 µm bis 100 µm, vorzugsweise im Bereich von 10 µm bis 50 µm. In bestimmten Ausführungsvarianten kann auch eine Schichtdicke bis zu 200 µm vorgesehen sein.

Für Anwendungen mit sehr geringen Platzverhältnissen genügt bereits das erste Coating, um eine möglichst hohe Biegefestigkeit zu gewährleisten.

Hier sei angemerkt, dass auch andere Verfahren denkbar sind, um insbesondere die Festigkeit der Faser zu erhöhen. So könnte beispielsweise durch gezielte Temperatur-Prozesse ähnlich dem thermischen Vorspannen von Glas oberflächennah eine höhere Druckvorspannung aufgebaut werden, was die Biegefestigkeit der Faser erhöhen kann. Denkbar ist auch das chemische Härten der Faser. Hierzu wäre allerdings, um die optischen Eigenschaften der Faser zu erhalten, ein weiterer Mantel notwendig, in dem durch Ionen-Austausch in einer salzschmelze oder durch Aufsprühen einer Salz-Schicht mit nachfolgender Temperung eine gezielte zusätzliche Druckvorspannung in diesem zusätzlichen Mantel aufgebaut werden kann. Ebenso denkbar wäre ein Elektronen- oder lonenstrahl-Härten. Allerdings sind all diese Verfahren vergleichsweise aufwendig. Zudem ist es schwierig, die optischen Eigenschaften der Fasern beizubehalten.

Die Beschichtung kann gemäß einer weiteren Ausführungsform auch lichtblockend ausgeführt sein, also opak oder lichtabsorbierend, beispielsweise farbig, wie schwarz oder blau, ausgeführt sein. Dies ist vorteilhaft, weil auf diese Weise ein Übersprechen auf den Kamerachip reduziert werden kann.

Eine Ausführungsform, bei welchem der Lichtleiter mindestens eine Glasfaser, insbesondere eine Glasfaser umfassend ein mehrkomponentiges silikatisches Glas oder eine Glasfaser aus einem mehrkomponentigen silikatischen Glas, umfasst oder bevorzugt als Glasfaserbündel, insbesondere als Glasfaserbündel umfassend Glasfasern umfassend ein mehrkomponentiges, silikatisches Glas oder aus einem mehrkomponentigen silikatischen Glas oder aus Glasfasern aus einem mehrkomponentigen silikatischen Glas, ausgebildet ist, ist dabei besonders vorteilhaft. Denn mit solchen Glasfasern sind die optischen Eigenschaften des diese Glasfasern umfassenden Glasfaserbündels und mithin des Lichtleiters bzw. des Endoskops besonders flexibel einstellbar. Weiterhin weisen solche Lichtleiter, die auf Glasfasern, basieren, eine deutlich höhere Temperaturbeständigkeit als eine polymere optische Faser (POF) auf. Dies ist insbesondere dann relevant, wenn eine besonders gute Einkoppeleffizienz realisiert werden soll, beispielsweise dann ein dünnes Faserbündel aus oder umfassend Glasfasern direkt auf einen LED-Chip kontaktiert werden oder sehr nahe an einen solchen Chip herangeführt werden. Eine polymere optische Faser bzw. ein Faserbündel aus oder umfassend polymere optische Fasern würde einer solchen thermischen Belastung jedoch nicht standhalten, sondern es würden zum Schmelzen der Fasern kommen.

Gemäß einer Ausführungsform ist die eine lichtleitende Faser oder sind die mehreren lichtleitenden Fasern am proximalen Ende in eine Einkoppelhülse gefasst, welche als mechanisches Interface zur Laser-Lichtquelle ausgeführt ist und damit eine definierte Lichteinkopplung hinsichtlich Fokusabstand und Zentrierung zur Lichtquelle ermöglicht. Bei einer Einzelfaser oder bei mehreren Einzelfasern, idealerweise drei oder sieben Einzelfasern, können beispielsweise sogenannte SMA-Stecker als Einkoppelhülse vorgesehen sein, die insbesondere eine definierte Ausrichtung zur Laser-Lichtquelle ermöglichen und auch insbesondere für Laser-Anwendungen gebräuchlich sind. Ebenso denkbar sind dafür auch sogenannte FC-Stecker. Hier ist insbesondere wieder eine Anordnung aus sieben Einzelfasern besonders vorteilhaft, da hier im Wesentlichen ein kreisrunder Querschnitt zu einen und zum anderen eine minimierte Zwickelfläche zwischen den Einzelfasern ermöglicht wird. Dies hat Vorteile hinsichtlich der Einkoppeleffizienz. Unter Zwickel versteht man die Zwischenräume bei einem Bündel aus kreisrunden Fasern. Eine weitere optimale Faseranordnung ergäbe sich bei 19 Einzelfasern, bei denen dann die Einzelfasern optimal dicht gepackt in zwei Schalen um eine Zentralfaser angeordnet sind. Die Fixierung der Einzelfasern erfolgt dabei i.d.R. mittels eines Klebers, z.B. eines 2-komponentigen heißvernetzenden Epoxy-Klebers oder mittels eines UV-aushärtenden Klebers.

Im Hinblick auf die eher übliche quadratische oder rechteckige Chip-Form von Laser- oder Leuchtdioden oder des Konverters als Lichtquelle kann es aber auch vorteilhaft sein, wenn vier Einzelfasern oder ganzzahlige Vielfache von zwei oder vier derartiger Fasern verwendet werden, derart, dass die Chip-Fläche möglichst gut abgedeckt wird. Einerseits können die für die Beleuchtung zur Verfügung stehenden Kavitäten hinsichtlich einer möglichst hohen aktiven Faserfläche, also der eigentlich lichtleitende Querschnittsbereich der Faser, mit mehr Fasern gefüllt werden, und anderseits eine bessere Lichteinkopplung erzielt werden.

Um die Einkoppeleffizienz zu steigern, kann zudem vorgesehen sein, dass die lichtleitenden Fasern am proximalen Ende heiß-verschmolzen angeordnet sind. Hierbei können Zwickelflächen zum einen minimierten werden, da durch den Heiß-Verformungsprozess die an sich runden Einzelfasern zu einer zumindest annähernden hexagonalen Querschnittsfläche verformt werden und sich damit nahezu lückenlos anordnen lassen. Zudem können bei vorgegebenen Einkoppelquerschnitt bzw. Fokusdurchmesser mehr Fasern untergebracht und damit ein höherer Lichtstrom übertragen werden.

Es ist möglich, dass solche heißverschmolzenen Fasern beispielsweise am proximalen Ende in einer Einkoppelhülse angeordnet sind. Es ist jedoch ebenfalls möglich, dass die heißverschmolzenen Fasern hülsenlos am proximalen Ende vorliegen. Dies ist insbesondere für Ausgestaltungen vorteilhaft, bei welchen eine effiziente Raumnutzung erforderlich ist, also beispielsweise bei besonders geringen Flächenquerschnitten am proximalen Ende o.ä.

Gemäß einer weiteren Ausführungsform ist die zumindest eine lichtleitende Faser und/oder sind die mehreren lichtleitenden Fasern und oder ist der Lichtleiter am distalen Ende gegenüber dem proximalen Ende verformt. Dies bedeutet, dass die zumindest eine lichtleitende Faser und/oder dass die mehreren lichtleitenden Fasern und/oder dass sogar der Lichtleiter selbst gemäß einer Ausführungsform eine Querschnittsfläche aufweisen kann, welche am distalen Ende eine andere Form aufweist als am proximalen Ende. Beispielsweise ist es möglich, dass die Querschnittsfläche der einen Faser und/oder auch der mehreren Fasern und/oder des Lichtleiters am proximalen Ende im Wesentlichen, also im Rahmen der Messgenauigkeit, rund ausgebildet sein kann, am distalen Ende aber beispielsweise oval oder nierenförmig vorliegt oder eine Querschnittsfläche aufweist, welche von mindestens zwei Linien begrenzt ist, die voneinander unterschiedliche Krümmungsradien aufweisen und/oder die als Differenzfläche von zwei einander nur teilweise überlappenden Kreisen und/oder Ellipsen ausgebildet ist. Insbesondere kann die Querschnittsfläche als Kreissegment ausgestaltet sein, wobei im Falle des Kreissegments ein Krümmungsradius unendlich ist, also eine im Rahmen der Messgenauigkeit gerade Linie ist. Eine solche Querschnittsfläche, welche als Kreissegment ausgebildet ist, kann auch als eine D-förmige Querschnittsfläche bezeichnet werden oder als eine im Wesentlichen D-förmige Querschnittsfläche.

Es ist auch möglich, dass unterschiedliche lichtleitende Fasern unterschiedliche Querschnittsflächen aufweisen, wobei insbesondere am proximalen Ende die Querschnittsfläche rund sein kann, im distalen Ende aber für eine oder mehrere Fasern oval, für andere nierenförmig. Es sind auch andere Querschnittsflächen denkbar, beispielsweise rechteckige oder annähernd rechteckige Querschnittsflächen, insbesondere am distalen Ende, oder allgemein polygonale Querschnittsflächen. Insbesondere eine Querschnittsfläche, welche beispielsweise als Kreissegment ausgebildet ist, bietet hier eine hohe Ausnutzung der zur Verfügung stehenden Kavitäten und kann somit zu einem erhöhten Lichtstrom bzw. zu einer erhöhten Beleuchtungsstärke am distalen Ende des Endoskops führen.

Eine solche Ausgestaltung kann insbesondere dann vorteilhaft sein, um eine räumliche besonders günstige Anordnung der Faser und/oder der Fasern und/oder des Lichtleiters in Bezug auf den Kamerachip zu gewährleisten.

Allgemein ist es möglich, dass die zumindest eine lichtleitende Faser und/oder dass die lichtleitenden Fasern zumindest abschnittsweise eine Querschnittsfläche aufweisen, welche von einer zumindest im Rahmen der Messgenauigkeit von der runden Form abweichende Form abweicht. Dies kann vorteilhaft sein, um besonders effiziente, beispielsweise raumsparende, Anordnungen einzelner Elemente im zweiten Bauteil des Endoskops zu ermöglichen.

Gerade im distalen Ende des Lichtleiters kann dies vorteilhaft sein.

Gemäß einer Ausführungsform weist daher die zumindest eine lichtleitende Faser und/oder weisen die mehreren lichtleitenden Fasern zumindest am distalen Ende des Lichtleiters eine Querschnittsfläche mit einer abgeflachten Form mit einem Aspektverhältnis von mindestens 1,5 : 1 und/oder eine ovale Querschnittsfläche und/oder eine nierenförmige Querschnittsfläche auf und/oder eine Querschnittsfläche, welche von mindestens zwei Linien begrenzt ist, die voneinander unterschiedliche Krümmungsradien aufweisen, und/oder die als Differenzfläche von zwei einander nur teilweise überlappenden Kreisen und/oder Ellipsen ausgebildet ist.

Wie bereits eingangs erwähnt, gestaltet sich insbesondere die Montage von dünnen Faserbündeln in ein Kamera-Endoskop als besonders schwierig und zeitaufwendig, was insbesondere mit wenigen dickeren lichtleitenden Fasern erheblich besser realisiert werden kann. Allerdings ergibt sich auch hier und allgemein, ohne Beschränkung auf die besondere Ausgestaltung eines Endoskops mit einem vereinfachten Einbau, insbesondere eines Endoskops umfassend einen Lichtleiter umfassend wenige, im Vergleich recht dicke Einzelfasern, das Problem, dass beim Zusammenbau der Kamera und beim Packen der Fasern um die Kamera herum, während die Kamera in der Mitte des distalen Gehäuses für die Kamera und die Fasern gehalten wird, dass sich die Kamera einseitig verschieben kann und dadurch ungleiche Hohlräume entstehen können, die eine definierte und gleichmäßige Anordnung der Fasern nicht mehr gewährleisten können. Dies führt dazu, dass sich auf einer Seite der Kamera mehr Fasern befinden und auf einer anderen Seite der Kamera weniger Fasern, was zu einer unausgewogenen Beleuchtung des zu untersuchenden Gewebes führen kann.

Ein weiterer Nachteil ist, dass die optischen Fasern Licht durch den Fasermantel der Faser emittieren können und die die Kamera an oder über deren Seitenflächen für dieses Streulicht empfindlich ist bzw. solches aufnimmt. Eine schlechte Bildqualität, z.B. zu geringer Kontrast, aber auch Bildartefakte können die Folge sein. Traditionell kann das Problem gelöst werden, wenn die Kamerawände mit einer lichtundurchlässigen Farbe oder einem opaken Epoxidharz lackiert oder vergossen werden, um das Eindringen von Streulicht zu reduzieren, und/oder dass die Fasern und die Kamera mit einem lichtundurchlässigen Klebstoff in das distale Gehäuse oder den Schaft der Geräte geklebt werden. Die Verwendung undurchsichtiger Klebstoffe schließt allerdings die Möglichkeit aus, UV- oder lichthärtende Klebstoffe für eine schnelle Montage zu verwenden. Ggf. schließt die vergleichsweise niedrige Temperaturbeständigkeit der Kamerachips die Verwendung von wärmeaushärtenden Klebstoffen aus. Daher werden typischerweise bei Raumtemperatur aushärtende Klebstoffe verwendet, die lange Aushärtezeiten haben und ein zusätzliches Verschieben der Kameraposition während der Aushärtezeit zur Folge haben können, was ebenfalls zu einer versetzten Positionierung der Kamera im distalen Gehäuse oder Schaft beiträgt. Zudem können dadurch häufig auch die lichtleitenden Fasern schief, d.h. nicht parallel ausgerichtet zueinander liegen, und daraus resultierend zu einer negativen Beeinflussung der Strahlausrichtung führen. Wenn Fasern nach der Montage geschliffen und poliert werden, so können schräge Endflächen entstehen, die bspw. nicht senkrecht zu ihrer optischen Achse ausgerichtet sind. Dies kann die Rückreflexion erhöhen, den Lichteinfall auf das Ziel verringern und dazu beitragen, dass das Licht auch vom beabsichtigten Ziel weggebrochen wird oder unerwünscht auch den Kamera Chip trifft.

Gemäß einem Aspekt der vorliegenden Offenbarung betrifft die vorliegende Anmeldung daher allgemein ein Endoskop umfassend einen Lichtleiter umfassend wenigstens eine lichtleitende Faser, ohne Beschränkung auf ein Endoskop mit einem Lichtleiter umfassend wenigstens eine lichtleitende Faser mit wenigstens einem Durchmesser von 80 µm, welches die vorgenannten Probleme des Standes der Technik zumindest teilweise mindert.

Gemäß einer Variante kann daher allgemein, ohne Beschränkung auf ein Endoskop mit einer vereinfachten Montage durch spezielle Ausgestaltung des Lichtleiters mit vorzugsweise nur wenigen, dafür relativ dicken Einzelfasern mit Durchmessern von 80 µm oder mehr, vorgesehen sein, dass das Endoskop eine Einhausung oder ein Einhausungselement umfasst oder aufweist. Die Einhausung oder das Einhausungselement kann eine Aufnahme für den Kamerachip aufweisen, um den Kamerachip zu positionieren. Vorzugsweise kann vorgesehen sein, den Kamerachip in der Mitte der Einhausung oder des Einhausungselements definiert zu positionieren. Vorzugsweise kann die Einhausung oder das Einhausungselement an der äußeren Seite ein oder mehrere Aufnahmeabschnitte zur definierten Aufnahme, Ausrichtung und/oder Lage der einen lichtleitenden Faser oder der mehreren lichtleitenden Fasern aufweisen, wobei besonders bevorzugt die Aufnahmeabschnitte einen, vorzugsweise trapezförmigen oder wannenförmigen, Querschnitt mit bevorzugt schräg verlaufenden Seitenwänden aufweisen. Mit anderen Worten kann das Endoskop gemäß einer Ausführungsform eine Einhausung oder ein weiteres, den Kamera-Chip bspw. bereits umfassendes Einhausungselement aufweisen, welches zum einen den Kamerachip in deren Mitte definiert positioniert und zum anderen an ihrer äußeren Seite ein oder mehrere Aufnahmeabschnitte zur definierten Aufnahme, Ausrichtung und/oder Lage der ein oder mehreren lichtleitenden Fasern aufweist, und wobei die Aufnahmeabschnitte einen vorzugsweise trapezförmigen bzw. wannenförmigen Querschnitt mit Seitenwänden, welche gemäß einer Ausführungsform schräg verlaufen, aufweisen. Damit können gleich mehrere der zuvor beschriebenen Probleme zumindest teilweise überwunden und optimalerweise sogar minimiert bzw. beseitigt werden. So können beispielsweise die Achsen der optischen Fasern parallel zur Kamera ausgerichtet montiert werden, wodurch die Schräglage der Fasern reduziert und ein Schräganschliff der Fasern vermieden werden kann. Insbesondere die vorzugsweise schräg verlaufenden Seitenwände der beispielsweise trapez- bzw. wannenförmigen Aufnahmeabschnitte ermöglichen dabei quasi eine Selbstjustage der Faserlage.

Gemäß einer Ausführungsform kann vorgesehen sein, für die Einhausung ein opakes bzw. dunkel, insbesondere schwarz eingefärbtes Material, zu verwenden. Dies ist vorteilhaft, da damit eine Streulichtbarriere zwischen lichtleitenden Fasern und Kamerachip erzielt werden kann. Die zuvor beschrieben negativen Auswirkungen bei der Bildqualität können damit weitgehend beseitigt werden. Vorteilhaft ist es weiterhin, wenn insbesondere ein Polymer-Material, welches sich bevorzugt spritzgießtechnisch herstellen lässt, für die Einhausung verwendet wird. Damit können zum einen geringe Kosten und zum anderen auch vergleichsweise komplexe Geometrien realisiert werden. Zudem kann zusätzliche noch eine zusätzliche elektrische Isolation realisiert werden. Beispiele für geeignete Materialien für eine solche Ausführungsform sind Polycarbonat (PC), ABS (Acrylnitril-Butadien-Styrol) und Polyamid (PA).

Gegenüber opak bzw. schwarz eingefärbten Klebern bei der traditionellen Verklebung der Fasern können damit gemäß der vorstehenden Ausführungsform nun auch transparente, insbesondere UV-aushärtende Kleber verwendet werden, was die Komplexität der Montage infolge kürzerer Aushärtezeiten reduzieren kann. Ein weiterer Vorteil ergibt daraus, dass die optischen Fasern zur distalen Kamerafläche etwas zurückgesetzt positioniert werden können, wobei diese dann nur gebrochen sein können und mit optisch klarer Vergussmasse, z.B. Epoxidharz oder UV-härtende Vergussmassen, vergossen sind, wodurch das Schleifen und Polieren der optischen Fasern entfallen kann und das Objektiv des Kamerachips nicht beeinträchtigt wird. Damit lässt sich zudem innerhalb des Gehäuses eine Zugentlastung für die empfindlichen Drähte oder die flexible Schaltung realisieren.

Hier sei angemerkt, dass derartige Ausführungsformen, bspw., eine Einhausung mit entsprechenden Aufnahmeabschnitten für die lichtführenden Fasern und/ weitere optische Komponenten bspw. die benannten Kameras oder auch optische Elemente zur Strahlführung und Formung oder auch die Integration einer oder mehrerer Lichtquellen je an distalen oder auch proximalen Ende und damit auch für andere Endoskope grundsätzlich mit den genannten Vorteilen zum Einsatz kommen kann, wobei letztendlich der verwendete Faserdurchmesser nur eine nebengeordnete Rolle spielt. Insbesondere ist es daher möglich und kann gemäß einer Ausführungsform nach diesem Aspekt auch vorgesehen sein, dass ein Lichtleiter umfassend wenigstens eine lichtleitende Faser mit einem Durchmesser zwischen 30 µm und 70 µm verwendet wird. Weiterhin ist es hier gemäß einer Ausführungsform auch möglich, dass der Lichtleiter als ein Faserbündel umfassend eine Vielzahl dünner Fasern mit Durchmessern zwischen 30 µm und 70 µm ausgebildet ist. Gemäß einer weiteren Ausführungsform ist die numerische Apertur der einen oder der mehreren lichtleitenden Fasern mindestens 0,7, bevorzugt mindestens 0,8 und besonders bevorzugt mindestens 0,85. Vorzugsweise umfasst der Kern der einen oder der mehreren lichtleitenden Fasern ein glasiges Material, dessen Zusammensetzung ausgewählt ist aus den vorstehend aufgeführten Glaszusammensetzungen und Glaszusammensetzungsbereichen für Kerngläser. Insbesondere kann der Kern der Glasfaser überwiegend, also zu mindestens 50 Gew.-%, oder im Wesentlichen, also zu mindestens 90 Gew.-%, oder sogar vollständig aus einem solchen glasigen Material bestehen.

Eine Ausführungsform, bei welcher der Kern der einen oder der mehreren lichtleitenden Fasern ein solches glasiges Material umfasst, ist vorteilhaft, weil auf diese Weise eine sehr gute Ausleuchtung des Kamera-Gesichtsfeldes (hier insbesondere für sogenannte C-MOS-Kameras mit z.B. 1 x 1 mm² Fläche) realisiert werden kann.

Gemäß einer weiteren Ausführungsform ist die eine lichtleitende Faser oder sind die mehreren lichtleitenden Fasern so ausgebildet, dass das Kern- und/oder das Mantelglas der einen lichtleitenden Faser oder der mehreren lichtleitenden Fasern bis auf unvermeidliche Spuren frei ist von Blei und/oder sonstigen Schwermetallen sowie frei von Antimon und/oder Arsen und/oder von sonstigen kritischen Elementen wie Cr(VI) ist.

In einer Ausführungsvariante der Erfindung kann vorgesehen sein, wie bereits zuvor beschrieben, dass die Lichtquelle als integraler Bestandteil der Steckverbindung ausgeführt, also in die Steckverbindung eingebaut, vorzugsweise fest in die Steckverbindung eingebaut, ist. Dabei kann vorgesehen sein, dass diese in der Steckverbindung eine Wärmesenke, z.B. in Form eines Metall-Formstücks, aufweist. Alternativ oder zusätzlich kann die Lichtquelle eine integrierte LED-Treiberschaltung, also eine in die Steckverbindung eingebaute, vorzugsweise fest eingebaute, Treiberschaltung, aufweisen. Dabei kann die integrierte LED oder die integrierte Treiberschaltung mittels elektrischer Kontakte mit dem ersten Bauteil des Endoskops verbindbar sein. Die Treiberschaltung kann dabei besonders vorteilhaft als platzsparender Vorwiderstand für die integrierte LED ausgeführt sein, welche noch eine zusätzliche Schutzdiode, z.B. eine Z-Diode, als Spannungsbegrenzung ausgeführt sein kann. Alternativ sind auch spezielle Strom-Konstantquellen als integrierte Schaltung einsetzbar, die eine fest vorgegeben konstanten Strom zum Betreiben der integrierten LED liefern. Besonders vorteilhaft ist, wenn bereits vom ersten Bauteil des Endoskops ein konstanter Strom über die elektrischen Kontakte zur integrierten LED eingespeist wird. Damit kann durch die nicht mehr benötigte integrierte LED-Treiberschaltung weiterer Bauraum in der Steckverbindung eingespart und auch die Verlustwärme minimiert werden, damit die integrierte LED mit einem möglichst hohen Strom betrieben werden kann, ohne die zulässigen Grenztemperatur an der Oberfläche der Steckverbindung zu überschreiten.

Im Rahmen der vorliegenden Offenbarung wird als integriert ein Bauteil verstanden, welches in ein anderes Bauteil eingebaut vorliegt, vorzugsweise fest eingebaut. Eine integrierte LED kann daher auch als eingebaute LED bezeichnet werden. In entsprechender Weise ist ein integraler Bestandteil eines weiteren Bauteils ein Bauteil, welches in das weitere Bauteil eingebaut, vorzugsweise fest eingebaut, vorliegt.

Als sehr effektive Lichtkopplung hat sich dabei erwiesen, wenn die Lichtkopplung zwischen der integrierten LED und der mindestens eine lichtleitende Faser des Lichtleiters als Stoßkopplung ausgeführt ist, wobei die mindestens eine lichtleitende Faser direkt mittels eines transparenten Klebers auf den Chip der integrierten LED aufgeklebt ist. Insbesondere ist dies vorteilhaft, wenn, wie zuvor beschrieben, die lichtleitende Faser eine sogenannte Weitwinkelfaser mit einer numerischen Apertur (NA) von mindestens 0,80 ausgeführt ist. Damit kann ein großer Anteil des von der LED abgestrahlten Lichtes eingefangen und zum distalen Ende des zweiten Bauteils geführt werden.

Bei den meisten Leuchtdioden ist der an sich lichtemittierende Chip durch eine Schicht vor direktem Kontakt mit der Umgebung geschützt oder eingegossen, wobei diese Schicht vielfältige Geometrien annehmen kann. Beispielsweise kann sie plan, aber auch gekrümmt zum Chip hin oder vom Chip weg ausgebildet sein, insbesondere auch linsen- oder kalottenförmig. Entsprechend wird unter Aufkleben der lichtleitenden Faser auch deren Anbringen an oder auf eben dieser Schicht verstanden.

Ein weiterer Aspekt der vorliegenden Erfindung betrifft ein Einweg-Endoskopsystem mit einem ersten Bauteil und steril einzeln verpackten zweiten Bauteilen, welche vorzugsweise als Schäfte ausgebildet sind oder sein können, welche nach der Entnahme aus ihrer sterilen Verpackung lösbar mit dem ersten Bauteil koppelbar sind, um ein Endoskop, insbesondere ein Endoskop nach Ausführungsformen der vorliegenden Offenbarung, zu erhalten.

Unter einem Schaft wird im Rahmen der vorliegenden Offenbarung ein zweites Bauteil eines Endoskops verstanden, welches einen im Vergleich zu seiner Länge nur geringe Querschnittsfläche aufweist. Mit anderen Worten ist der Schaft also im Vergleich zur Länge dünn ausgebildet. Eine solche Ausgestaltung eines zweiten Bauteils als Schaft ist vorteilhaft, gerade wenn nur sehr schwer zugängliche Bereiche mittels des Endoskops untersucht werden und/oder für Anwendungen in der Medizintechnik.

Ein Vorteil des Endoskop-Systems nach der vorliegenden Offenbarung ist es, für kurz aufeinander abfolgende Untersuchungen bereits steril verpackte zweite Bauteile, insbesondere Schäfte, vorliegen zu haben, so dass rasche mehrere Bereiche untersucht werden können oder, bei medizinischen Untersuchungen, mehrere Untersuchungen von unterschiedlichen Patienten kurz hintereinander unter Sicherstellung einer ausreichenden Hygiene durchgeführt werden können. Daher ist es gerade für das Endoskop-System nach der vorliegenden Offenbarung vorteilhaft, dass die zweiten Bauteile lösbar mit dem ersten Bauteil koppelbar sind, um auf diese Weise die Vorteile eines Einweg-Endoskops zu ermöglichen, wobei gleichzeitig durch die Teile des Endoskopsystems, welche beispielsweise bei medizinischen Untersuchungen oder sonstigen medizinischen Einsatzzwecken nicht zwingend sterilisiert vorliegenden müssen, in einem mehrfach nutzbaren, ersten Bauteil untergebracht sind.

Gemäß einer Ausführungsform ist das zweite Bauteil als zumindest abschnittsweise flexibler Schaft vorgesehen, welcher eine flexible Ummantelung mit einem Schlauch oder Geflechtschlauch oder Schrumpfschlauch umfasst, welche den Lichtleiter mit seiner mindestens einen lichtleitenden Faser sowie eine Versorgungsleitung zur elektrischen Speisung des Kamerachips und vorzugsweise mindestens eine Rücksignalleitung zu einer Daten- und/oder Bildweiterverarbeitungseinheit, welche insbesondere als Bestandteil im ersten Bauteil vorliegen kann, zumindest abschnittsweise umschließt. Eine solche Ausgestaltung, insbesondere mit einem flexiblen Schaft, ist insbesondere für medizinische Anwendungen geeignet.

Gemäß einer weiteren Ausführungsform ist das zweite Bauteil ausgebildet als zumindest abschnittsweise starrer Schaft vorgesehen, welcher eine starre Ummantelung mit einer Hülse umfasst, welche den Lichtleiter mit seiner mindestens einen lichtleitenden Faser sowie eine Versorgungsleitung zur elektrischen Speisung des Kamerachips und vorzugsweise eine Rücksignalleitung, vorzugsweise zu einer Daten- und/oder Bildweiterverarbeitungseinheit, welche insbesondere als Bestandteil im ersten Bauteil vorliegen kann, umschließt. Eine solche Ausgestaltung kann insbesondere vorteilhaft sein, weil auf diese Weise die vom zweiten Bauteil, das hier als starr ausgebildeter Schaft vorgesehen ist, umfassten Elemente besser gegenüber mechanischen Belastungen geschützt sein können.

### Beschreibung der Zeichnungen

Im Folgenden wird die Erfindung anhand von Figuren weiter erläutert. Dabei bezeichnen gleiche Bezugszeichen die gleiche oder einander entsprechende Elemente. Es zeigen:
- Fig. 1: eine schematische und nicht maßstabsgetreue Abbildung eines Endoskops nach einer Ausführungsform,
- Fig. 2: ein schematischer Ausschnitt eines Endoskops gemäß einer weiteren Ausführungsvariante
- Fig. 3a bis 3e: schematische und nicht maßstabsgetreue Abbildungen von distalen Enden eines Endoskops.
- Fig. 4 und 5: in einem 3D-CAD-Modell das distale Ende eines Endoskops mit einem Kamerachip und einer Einhausung für den Kamerachip

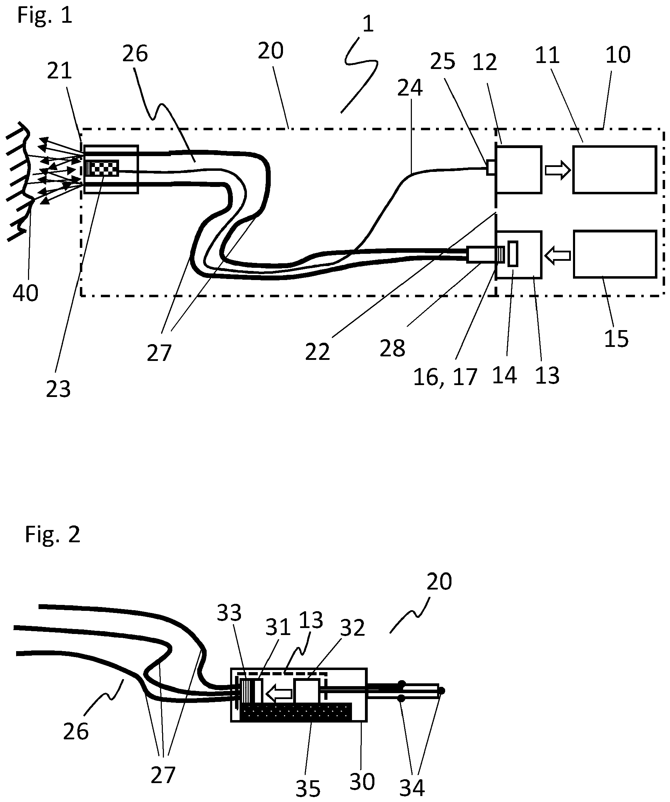

Fig. 1 ist eine schematische und nicht maßstabsgetreue Abbildung eines Endoskops 1 nach einer Ausführungsform. Das Endoskops 1 umfasst eine erstes Bauteil 10 und ein zweites Bauteil 20, wobei hier in der Darstellung das erste Bauteil 10 rechts angeordnet ist und das zweite Bauteil 20 links. Das zweite Bauteil 20 umfasst ein mit dem ersten Bauteil 10 verbundenes proximales Ende 22. Es kann vorgesehen sein, dass das mit dem ersten Bauteil 10 verbundene proximale Ende 22 des zweiten Bauteils 20 so ausgestaltet ist, dass es lösbar ist. Insbesondere können also die beiden Bauteil 10 und 20 so ausgestaltet sein, dass sie mittels einer lösbaren Verbindung ausgestaltet vorliegen. Dies kann insbesondere dann von Vorteil sein, wenn ein Bauteil lediglich für einen Einmalgebrauch vorgesehen ist, das andere Bauteil, hier beispielsweise das erste Bauteil 10, Komponenten umfasst, welche für den Mehrfachgebrauch bestimmt sind, insbesondere solche, welche hochwertig und/oder hochpreisig sind. Insbesondere kann dies der Fall sein, wenn eine besondere Lichtquelle, wie beispielsweise eine Lichtquelle umfassend mindestens einen Laser, von einem der Bauteile, hier beispielsweise dem ersten Bauteil 10, umfasst ist. Dies gilt insbesondere auch für eine Daten- und/oder Bildverarbeitungseinheit 11 sowie einer hochwertigen Lichtquelle 13 mit Hochleistungs-LED-Chip 14 und deren LED-Treiberschaltung 15 und dem nicht dargestellten Netzteil, was ebenfalls Bestandteil des ersten Bauteils 10 sein kann.

Das zweite Bauteil 20 weist weiterhin ein distales Ende 21 auf, wobei im distalen Ende 21 ein Kamerachip 23 zur Bildaufnahme einer Gewebeoberfläche 40 angeordnet ist. Im zweiten Bauteil 20 verläuft weiterhin ein Lichtleiter 26 umfassend mindestens eine lichtleitende Faser 27. Diese ist ausgestaltet, um Licht einer Lichtquelle 13 vom proximalen Ende 22 zum distalen Ende 21 des Lichtleiters 26 zu leiten und am distalen Ende 21 abzugeben, um die Gewebeoberfläche 40 möglichst hell und gleichmäßig auszuleuchten. Weiterhin verläuft im zweiten Bauteil 20 eine Versorgungsleitung (nicht dargestellt) zur elektrischen Speisung des Kamerachips 23.

Die Lichtquelle 13 umfasst mindestens einen LED-Chip 14, der über eine LED-Treiberschaltung mit einem Strom versorgt wird. Zudem kann an seinem optischen Interface 16, welches zur Aufnahme einer optischen Steckverbindung 28 des zweiten Bauteils 20 dient, ein optisches Element 17 in Form einer Linse oder einer Linsenanordnung vorgesehen sein, um z.B. das Licht des LED-Chips 14 in das proximale Ende 22 des Lichtleiters 26 in die lichtleitenden Fasern 27 einzukoppeln.

Insbesondere unter Montagegesichtspunkten, gerade wenn das zweite Bauteil 20 nur für den Einzelgebrauch vorgesehen ist, kann es vorteilhaft sein, wenn der Lichtleiter 26 höchstens zwanzig, vorzugsweise höchstens zehn lichtleitende Faser 27 aufweist. Es ist aber allgemein möglich, dass bis zu einigen hundert Einzelfasern 27 in einem Lichtleiter 26 sind, wobei dies von den entsprechenden Faserdurchmessern und der resultierenden bzw. adressierten Dicke des Faserbündels und mithin des Lichtleiters 26 abhängt und entsprechend die Zahl der Fasern 27 ausgewählt werden kann.

Typische Faserdurchmesser (oder Faserdicken) von lichtleitenden Fasern 27 können in bevorzugter Weise in einem Bereich von 100 µm bis 1000 µm, bevorzugt bis 600 µm, besonders bevorzugt bis 500 µm, liegen, wobei der maximale Faserdurchmesser vorzugsweise in einem Bereich von 100 µm bis 400µm liegt. Es sind aber auch dünnere Fasern mit Durchmessern von 30 µm, 50 µm oder 70 µm denkbar.

Gemäß einer Ausführungsform ist die eine lichtleitende Faser 27 oder sind die mehreren lichtleitenden Fasern 27 als Stufenindex-Glasfasern ausgebildet.

Vorzugsweise kann die eine lichtleitende Faser 27 und/oder können die mehreren lichtleitenden Fasern 27 so ausgebildet sein, dass die numerische Apertur (NA) gegen Luft der mindestens einen Faser 27 und/oder der mehreren lichtleitenden Fasern 27 mindestens 0,7, bevorzugt mindestens 0,8 und besonders bevorzugt mindestens 0,85 ist. Dies kann besonders günstig sein, um einen hohen CRI (Color rendering index) zu erhalten und aber vor allem einen möglichst hohen Lichtstrom am distalen Ende 21 zu erzielen.

Insbesondere unter Montagegesichtspunkten kann es günstig sein, wenn die mindestens eine lichtleitende Faser 27 oder die mehreren lichtleitenden Fasern 27 am proximalen Ende 22 des Lichtleiters 26, wie in Fig. 1 schematisch auch dargestellt, in einer optischen Steckverbindung 28, z.B. in Form einer Einkoppelhülse angeordnet sind.

Das zweite Bauteil 20 kann beispielsweise als zumindest abschnittsweise flexibler Schaft vorgesehen sein oder auch als zumindest abschnittsweise starrer Schaft. Das zweite Bauteil kann beispielsweise eine Ummantelung umfassen. Für den Fall, dass das zweite Bauteil 20 als zumindest abschnittsweise flexibler Schaft ausgebildet ist, ist die Ummantelung flexible ausgestaltet, insbesondere mit einem Schlauch oder Geflechtschlauch oder mit einem Schrumpfschlauch. Für den Fall der Ausbildung des zweiten Bauteils 20 als zumindest abschnittsweise starrer Schaft ist die Ummantelung vorzugsweise starr ausgebildet und umfasst eine Hülse oder einen Rohrabschnitt. Allgemein, ohne Beschränkung auf das hier beispielhaft dargestellte Beispiel, umschließt die Ummantelung den Lichtleiter 26 mit der mindestens einen Faser 27, eine Versorgungsleitung zur elektrischen Speisung des Kamerachips 23 und vorzugsweise mindestens eine Rücksignalleitung 24, vorzugsweise eine Leitung zu einer Daten- und/oder Bildweiterverarbeitungseinheit 11, welche insbesondere als Bestandteil im ersten Bauteil 10 vorliegen kann, zumindest abschnittsweise.

Als ein besonders bevorzugtes Ausführungsbeispiel erweist sich eine Anordnung mit sieben ca. 200 µm dicken lichtleitenden Fasern 27, welche als sogenannte Weitwinkelfaser mit einer NA > 0,85 ausgeführt sind, wobei die sieben lichtleitenden Fasern 27 um den Kamerachip 23 angeordnet sind, und am proximalen Ende 22 in einer optischen Steckverbindung 28 verklebt sind. Alternativ können diese sieben lichtleitenden Fasern 27 in der optischen Steckverbindung 28 auch heiß-verschmolzen sein. Allgemein ist es aber auch möglich und kann sogar bevorzugt sein, wenn am proximalen Ende heiß-verschmolzene Fasern hülsenlos vorliegen.

Fig. 2 zeigt ebenfalls schematisch einen Ausschnitt des zweiten Bauteils des Endoskops 20 bei dem die Lichtquelle 13 integraler Bestandteil einer Steckverbindung 30 ist, welche das proximale Ende des zweiten Bauteils 22 mit seinem Lichtleiter 26 bildet und wobei die Lichtquelle 13 mindestens eine integrierte LED 31 aufweist, die ihr abgegebenes Licht in die zumindest eine lichtleitende Faser 27 des Lichtleiters 26 einkoppelt.

Die Lichtquelle 13, als integraler Bestandteil der Steckverbindung 30 ausgeführt, kann zusätzlich eine Wärmesenke 35 und/oder eine integrierte LED-Treiberschaltung 32 aufweisen, wobei die integrierte LED 31 oder die integrierte Treiberschaltung 32 mittels elektrischer Kontakte 34 mit dem ersten Bauteil des Endoskops 10 verbindbar ist.

Als durchaus effiziente Lichtkopplung hat sich erwiesen, wenn die Lichtkopplung zwischen der integrierten LED 31 und der mindestens eine lichtleitenden Faser 27 des Lichtleiters 26 als Stoßkopplung ausgeführt ist, wobei die mindestens eine lichtleitende Faser 27 direkt mittels eines transparenten Klebers auf den Chip der integrierten LED 31 stirnseitig aufgeklebt ist. Zusätzlich kann es aber auch erforderlich sein, wenn ein optisches Element 33 in Form einer einfachen Linse oder Linsenanordnung das Licht derart kollimiert, dass möglichst viel Licht in die Faser 27 eingekoppelt werden kann. Insbesondere bei Verwendung von weitwinkligen Fasern 27 mit hohem Akzeptanzwinkel (> 100°) führt aber eine direkte Stoßkopplung oft zu deutlich besseren Ergebnissen, zumal Fresnel-Verluste beim Übergang weiterer Grenzflächen, hier an dem optischen Element 33, vermieden werden können.

Allerdings kann es auch von Vorteil sein, wenn das optische Element 33 als dünne transparent Schicht, z.B. als Kunststoff-Folie oder als Dünnglas, ausgeführt ist. Dies verhindert insbesondere mechanische Beschädigungen der oft hauchdünnen Konverterschicht bei den LED-Chips der integrierten LED 31. Eine solche Schicht kann sich insbesondere auch als wirksamer mechanischer Schutz bei dem in Figur 1. dargestellten Ausführungsbeispiel zeigen. Hier könnte beispielsweise das optische Element 17 als Kunststoff-Folie oder als Dünnglas ausgeführt sein. Insbesondere in diesem Ausführungsbeispiel, bei dem die optische Steckverbindung 28 des zweiten Bauteils 20 häufig in das optische Interface 16 oder aus diesem ausgesteckt wird, kann damit der LED-Chip 14 mechanisch geschützt werden.

Als LED-Chips, sowohl für die Variante, bei der der LED-Chip im ersten Bauteil des Endoskops verbaut ist oder aber für die Variante, bei der ein integrierter LED-Chip in der Steckverbindung des zweiten Bauteils verbaut ist eignen sich insbesondere leistungsstarke kompakte Weißlicht-LEDs, welche bei einer Chip-Abmessung von 1 mm² sehr hohe Lumenwerte pro Watt erzeugen können. Insbesondere mit dieser vergleichsweise kleinen Chip-Fläche lässt sich eine hohe Koppeleffizienz zu den lichtleitenden Fasern realisieren. Beispiele für solche LEDs sind die LUMILEDS LUXEON Z LXZ1-4080, welche einerseits einen CRI von 80 ermöglicht und anderseits bei 1,4 W einen Lichtstrom von 130 Im erzeugt bei einer Chipgröße von 1 mm². Ein anderes Beispiel ist die OSRAM OSLON Pure 1010, welche einen CRI von 90 ermöglicht und bei 0,98 W einen Lichtstrom von 95 Im erzeugt.

Mit derartigen LEDs lassen sich auch noch insbesondere bei der Variante, bei der die LED in der Steckverbindung integriert ist, ausreichend große Im-Werte erzielen, wenn diese hinsichtlich der Einhaltung einer maximal zulässigen Oberflächentemperatur an der Steckverbindung hinsichtlich des Stroms und damit der Leistung herunter geregelt betrieben werden müssen.

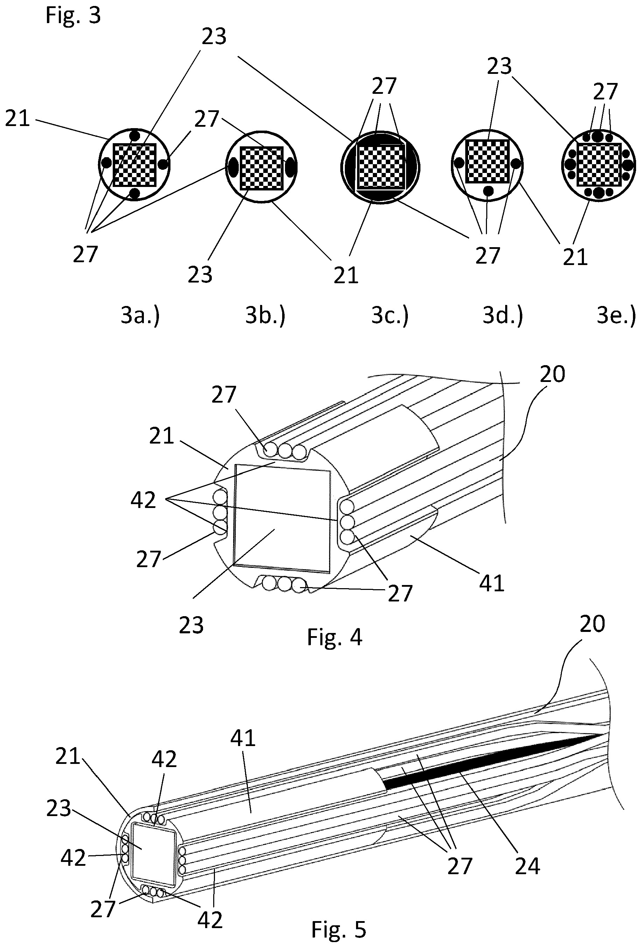

Fig. 3a bis 3e zeigen schematisch und nicht maßstabsgetreu Abbildungen von distalen Enden 21 eines zweiten Bauteils 20 eines Endoskops 1. Das distale Ende 21 umfasst jeweils den Lichtleiter 26 umfassend hier jeweils mehrere lichtleitende Fasern 27 sowie jeweils einen Kamerachip 23.

In der linken Darstellung von Fig. 3 (Fig. 3a) sind vier Fasern 27 angeordnet, welche einen im Rahmen der Messgenauigkeit runden Querschnitt aufweisen. Diese sind hier so um den Kamerachip 23, welcher hier beispielhaft als eine annähernd quadratische Form aufweist, angeordnet, dass jeweils an einer Seite des Kamerachips 23 eine Faser 27 ist. In der Darstellung von Fig. 3d sind hingegen lediglich auf drei Seiten des Kamerachips 23 Fasern 27 angeordnet.

In der Darstellung der Fig. 3b sind lediglich zwei Fasern 27 auf zwei Seiten des Kamerachips 23 angeordnet. Hier ist der Querschnitt der lichtleitenden Fasern 27 nicht rund, sondern vielmehr oval bzw. elliptisch ausgebildet. Insbesondere können die lichtleitenden Fasern 27 dabei so ausgebildet sein, dass sie am distalen Ende 21, wie hier, gegenüber dem proximalen Ende 22 - hier nicht dargestellt - verformt sind. Insbesondere ist es möglich, dass die lichtleitenden Fasern 27 am proximalen Ende 22 einen runden Querschnitt aufweisen, am distalen Ende aber, wie hier, verformt vorliegen. Dies kann vorteilhaft sein, um die Fasern 27 um den Kamerachip 23 herum anzuordnen.

Vorzugsweise können die lichtleitenden Fasern 27 und/oder die zumindest eine lichtleitende Faser 27 zumindest am distalen Ende 21, wie hier beispielhaft dargestellt, eine Querschnittsfläche mit einer abgeflachten Form, insbesondere mit einem Aspektverhältnis von mindestens 1,5 : 1 und/oder eine ovale Querschnittsfläche und/oder eine nierenförmige Querschnittsfläche auf und/oder eine Querschnittsfläche, welche von mindestens zwei Linien begrenzt ist, die voneinander unterschiedliche Krümmungsradien aufweisen, und/oder die als Differenzfläche von zwei einander nur teilweise überlappenden Kreisen und/oder Ellipsen ausgebildet ist. Andere Querschnittsformen, beispielsweise Polygone, sind denkbar, jedoch sind gerade abgeflachte Formen besonders vorteilhaft hinsichtlich der Anordnung der lichtleitenden Fasern 27 um den Kamerachips 23 herum. Fig. 3c zeigt eine Anordnung, bei denen 4 Fasern, welche eine Querschnittsfläche, die hier als Kreissegment ausgebildet ist, am distalen Ende aufweisen, um den Kamerachip 23 herum angeordnet sind. Allgemein, ohne Beschränkung auf das hier konkret dargestellte Beispiel von Querschnittsflächen, welche als Kreissegment, hier also beispielsweise auch beschrieben werden können als "D", sind Querschnittsflächen möglich und vorteilhaft, welche von mindestens zwei Linien begrenzt sind, die voneinander unterschiedliche Krümmungsradien aufweisen und/oder die als Differenzfläche von zwei einander nur teilweise überlappenden Kreisen und/oder Ellipsen ausgebildet sind. Beispielsweise sind allgemein möglich sichelförmige Querschnittsflächen.

Sowohl die in Fig 3b als auch die in Fig. 3c dargestellten distalen Enden der Fasern 27 können beispielsweise durch einen Heiß-Umformprozess derart verformt werden. Dabei wird die Faser 27 über deren Verarbeitungstemperatur in einer Form aufgeheizt und dann unter Druck verformt. Bedingt durch die Viskosität des Fasermaterials lassen sich natürlich nicht perfekte Geometrien nachbilden. So wird eine Querschnittsfläche in Form eines Kreissegments, welche auch als "D-förmig" beschrieben werden kann, an den spitz zulaufenden Ecken kleinere Verrundungen aufweisen. Grundsätzlich kann eine derartige Formgebung sowohl bei Glasfaser, Quarz-Fasern oder auch Kunststofffasern angewendet werden, wobei die Umformtemperatur dem jeweiligen Material anzupassen ist. Bei Kunststofffasern (POFs) beträgt diese typischerweise 150° C bis 300° C, bei Glasfasern typischerweise zwischen 500° C und 800° C, je nach Glas-Type, und bei Quarz-Fasern bis zu 2000° C.

Fig.3e zeigt eine 12er-Anordnung, wie bereits zuvor beschrieben wurde. Hier sind insgesamt vier dickere Fasern 27 mit acht dünneren Fasern derart gruppiert, dass pro Kavität (Segment) die dicke Faser 27 in der Mitte der Kavität und die beiden dünneren Fasern 27 jeweils rechts und links von der dicken Faser 27 angeordnet ist. Damit kann trotz der vergleichsweise wenigen Fasern 27 bereits eine gute Flächennutzung der Kavität und damit ein vergleichsweise hoher Lichtstrom erzielt werden. Derartige Beispiele lassen sich z.B. auch auf eine 20er-Anordng mit 20 einzelnen Fasern 27, d.h. 5 Fasern 27 je Kavität ausweiten, bei denen es idealerweise 3 Durchmesserabstufungen bei den Fasern 27 gibt.

Fig. 4 zeigt in einem 3D-CAD-Modell das distale Ende 21 eines zweiten Bauteils des Endoskops 20 gemäß einer Ausführungsform mit einer Zwölfer-Anordnung von gleich dicken lichtleitenden Fasern 27, welche um einen Kamerachip 23 angeordnet sind. Gemäß einer Ausführungsform weist das Endoskop, ohne Beschränkung auf das dargestellte Beispiel, eine Einhausung 41 auf, welche eine Aufnahme für den Kamerachip 23 aufweist, um die Chip 23 in seiner Lage zu fixieren. Diese Einhausung kann beispielsweise so ausgestaltet sein, dass sie für die lichtleitenden Fasern 27 entsprechende Aufnahmeabschnitte 42 aufweist, mit denen die lichtleitenden Fasern 27 hinsichtlich ihrer Position und Winkel-Ausrichtung definiert angeordnet werden können. Dazu sind die Aufnahmeabschnitte gemäß der in Fig. 4 dargestellten Ausführungsform wannen- bzw. trapezförmig ausgestaltet. Dies hat den Vorteil, dass sich die Fasern 27 entsprechend bereits weitgehend parallel angeordnet platzieren können. Schräg und damit auch schräg geschliffene Fasern 27 mit den damit verbundenen Nachteilen der Abstrahlung am distalen Ende 21 können durch eine derartige Ausgestaltung der Einhausung 41 und der Aufnahmeabschnitte 42 vermieden werden. Zudem kann damit der Montageaufwand reduziert werden.

Fig. 5 zeigt die gleiche Anordnung wie in Fig. 4 dargestellt eingebaut in einem starren Schaft eines zweiten Bauteils 20 eines Endoskops. Zu den in Fig. 4 beschrieben Komponenten ist zu sehen, dass die Fasern 27 weitgehend parallel in den Aufnahmeabschnitten 42 der Einhausung 41 geführt sind.

Ergänzend sei angemerkt, dass die Einhausung 41 teil- oder abschnittsweise von einer starren oder flexiblen Hülle umgeben sein kann, um bspw. eine weitere Fixierung der Lichtleitfaser in den Aufnahmeabschnitten 42 zu unterstützen und die weitere Montage nochmals zu vereinfachen. Insbesondere bei der Verwendung weniger relativ dicker Faser ist auch denkbar die Einhausung 41 und die Hülle einstückig auszubilden, so dass die Lichtleitfaser in die so vorhandenen, meist schlitzförmigen Abschnitte oder Ausbrüche eingebracht werden können.

Idealerweise ist im Hinblick auf eine Abschirmung des Kamerachips 23 vor seitlichem Fremdlicht aus den lichtleitenden Fasern 27 die Einhausung 41 aus einem opaken, lichtundurchlässigen Material gefertigt. Besonders geeignet sind dazu Spritzgussteile aus einem schwarz eingefärbten Kunststoff, beispielsweise aus oder umfassend Polycarbonat (PC) und/oder Polyamid (PA). Zudem kann zur Faserfixierung vorgesehen sein, einen opaken, z.B. ebenfalls schwarz eingefärbten, Kleber zu verwenden. Allerdings erlaubt aber eine Einhausung 41 aus z. B. schwarzem Kunststoff die Verwendung auch von transparenten, z.B. UV-härtenden Klebern, was den Herstellprozess verkürzt und damit auch Kostenvorteile mit sich bringt.

Weiterhin dargestellt und bezeichnet ist die Rücksignalleitung 24.

### Bezugszeichenliste

- 1: Endoskop
- 10: erstes Bauteil des Endoskops
- 11: Daten- und/oder Bildverarbeitungseinheit
- 13: Lichtquelle
- 14: LED-Chip
- 15: LED-Treiberschaltung
- 16: optisches Interface
- 17: optisches Element
- 20: zweites Bauteil des Endoskops
- 21: distales Ende des zweiten Bauteils
- 22: proximales Ende des zweiten Bauteils
- 23: Kamerachip
- 24: Rücksignalleitung
- 25: elektrische Steckverbindung
- 26: Lichtleiter
- 27: lichtleitende Fasern
- 28: optische Steckverbindung
- 30: Steckverbindung
- 31: integrierte LED
- 32: integrierte LED-Treiberschaltung
- 33: optisches Element
- 34: elektrische Kontakte
- 35: Wärmesenke
- 40: Gewebeoberfläche
- 41: Einhausung
- 42: Aufnahmeabschnitt

## Patentansprüche

1. Endoskop (1) mit einem ersten Bauteil (10) und einem zweiten Bauteil (20),
wobei das zweite Bauteil (20) ein mit dem ersten Bauteil (10) verbundenes proximales Ende (22) und ein distales Ende (21) aufweist, wobei im distalen Ende (21) ein Element zur Bildaufnahme angeordnet ist, und wobei im zweiten Bauteil (20) ein Lichtleiter (26) mit zumindest einer lichtleitenden Faser (27) verläuft, um Licht der Lichtquelle (13) vom proximalen Ende (22) zum distalen Ende (21) zu leiten und am distalen Ende (21) abzugeben,
- wobei im ersten Bauteil (10) eine Lichtquelle (13) eingebaut ist, wobei die Lichtquelle (13) mindestens einen LED-Chip (14) zur Abgabe von Licht umfasst, wobei der LED-Chip (14) so mit dem am ersten Bauteil (10) verbundenen proximalen Ende (22) des zweiten Bauteils (20) gekoppelt ist, dass das vom LED-Chip (14) abgegebene Licht in die zumindest eine lichtleitende Faser (27) des Lichtleiters (26) einkoppelbar ist und wobei die mindestens eine lichtleitende Faser (27) einen Durchmesser von mindestens 80 µm aufweist und wobei die mindestens eine lichtleitende Faser (27) eine zumindest teil- und/oder abschnittsweise an der Mantelfläche angeordnete polymerbasierte Beschichtung und/oder eine Schutzhülle aus einem polymerbasierten Schlauchmaterial aufweist;
**dadurch gekennzeichnet, dass**
die wenigstens eine lichtleitende Faser (27) eine Glasfaser ist.

2. Endoskop (1) nach Anspruch 1, wobei der Lichtleiter (26) höchstens zwanzig lichtleitende Fasern (27) aufweist.

3. Endoskop (1) nach einem der Ansprüche 1 oder 2, wobei die eine lichtleitende Faser (27) oder die mehreren lichtleitenden Fasern (27) einen Durchmesser im Bereich von 100 µm bis 1000 µm aufweisen, wobei die lichtleitenden Einzelfasern (27) auch unterschiedliche Durchmesser aufweisen können.

4. Endoskop (1) nach einem der Ansprüche 1 bis 3, wobei die eine lichtleitende Faser (27) oder die mehreren lichtleitenden Fasern (27) Stufenindex-Glasfasern sind.

5. Endoskop (1) nach einem der Ansprüche 1 bis 4, wobei die numerische Apertur (NA) gegen Luft der einen oder der mehreren lichtleitenden Fasern (27) mindestens 0,7 ist.

6. Endoskop (1) nach einem der Ansprüche 1 bis 5, wobei der Lichtleiter (26) mehrere lichtleitende Fasern (27) umfasst, wobei wenigstens eine lichtleitende Faser (27), eine zumindest teil- und/oder abschnittsweise an deren Mantelfläche angeordnete polymerbasierte Beschichtung und/oder eine Schutzhülle aus einem polymerbasiertem Schlauchmaterial aufweist.

7. Endoskop (1) einem der Ansprüche 1 bis 6, wobei die Beschichtung eine Acrylat-, Polyurethan-, Polyimid-, Epoxi-, Polyamid-, Ethylen-Tetrafluorethylen-Copolymer- oder Poly-Xylylen-basierte Verbindung oder eine Mischung einer oder mehrere dieser Verbindungen umfasst oder aus einer solchen Verbindung oder einer Mischung einer oder mehrere dieser Verbindungen besteht.

8. Endoskop (1) nach einem der Ansprüche 1 bis 7, wobei die Beschichtung mittels Tauchen, Besprühen, Extrudieren oder Abscheidung bei niedrigem Druck auf die eine oder die mehreren lichtleitenden Fasern (27) aufbringbar oder aufgebracht ist.

9. Endoskop (1) nach einem der Ansprüche 1 bis 8, wobei die Beschichtung eine Dicke zwischen mindestens 5 µm und höchstens 100 µm aufweist.

10. Endoskop nach einem der Ansprüche 1 bis 9, aufweisend eine weitere äußere Beschichtung, welche bevorzugt PMMA, Polyamid (NYLON), Polyimid oder ein fluoriertes Polymer oder ein thermoplastisches Elastomer und/oder Polynivylidenfluorid oder Polytetrafluoroethylen oder auch Polyurethan oder Mischungen hiervon umfasst.

11. Endoskop (1) nach einem der Ansprüche 1 bis 10, wobei die eine lichtleitende Faser (27) oder die mehreren lichtleitenden Fasern (27) am proximalen Ende (22) des zweiten Bauteils in einer als Einkoppelhülse ausgeführten optischen Steckverbindung (28) angeordnet sind.

12. Endoskop (1) nach einem der Ansprüche 1 bis 11, wobei die lichtleitenden Fasern (27) am proximalen Ende (22) heiß-verschmolzen angeordnet sind.

13. Endoskop nach einem der Ansprüche 1 bis 12, wobei die zumindest eine lichtleitende Faser (27) und/oder die mehreren lichtleitenden Fasern (27) und/oder der Lichtleiter (26) am distalen Ende (21) gegenüber dem proximalen Ende (22) verformt ist.

14. Endoskop nach einem der Ansprüche 1 bis 13, wobei zumindest eine lichtleitende Faser (27) und/oder die mehreren lichtleitenden Fasern (27) zumindest am distalen Ende (21) des Lichtleiters (26) einen Querschnitt mit einer abgeflachten Form mit einem Aspektverhältnis von mindestens 1,5: 1 und/oder eine ovale Querschnittsfläche und/oder eine nierenförmige Querschnittsfläche aufweist und/oder eine Querschnittsfläche, welche von mindestens zwei Linien begrenzt ist, die voneinander unterschiedliche Krümmungsradien aufweisen, und/oder die als Differenzfläche von zwei einander nur teilweise überlappenden Kreisen und/oder Ellipsen ausgebildet ist.

15. Endoskop (1) nach einem der Ansprüche 1 bis 14, wobei die Lichtquelle (13) als in die Steckverbindung (30) eingebaut ausgeführt sein und/oder eine Wärmesenke (35) und/oder eine eingebaute LED-Treiberschaltung (32) aufweisen kann, und wobei die eingebaute LED (31) oder die fest eingebaute Treiberschaltung (32) mittels elektrischer Kontakte (34) mit dem ersten Bauteil (10) des Endoskops (1) verbindbar ist.

16. Endoskop (1) nach einem der Ansprüche 1 bis 15, wobei die Lichtkopplung zwischen der eingebauten LED (31) und der mindestens einen lichtleitenden Faser (27) des Lichtleiters (26) als Stoßkopplung ausgeführt ist, wobei die mindestens eine lichtleitende Faser (27) direkt mittels eines transparenten Klebers auf den Chip der eingebauten LED (31) aufgeklebt ist.

17. Endoskop (1), nach einem der Ansprüche 1 bis 16, weiter umfassend eine Einhausung (41), welche auf eine Aufnahme für den Kamerachip (23) aufweist, um den Kamerachip (23) zu positionieren.

18. Endoskop (1) nach Anspruch 17, wobei die Einhausung (41) so ausgeführt ist, dass sie ein opakes oder dunkel, eingefärbtes Material umfasst oder aus einem opaken oder dunkel eingefärbten, Material, ausgeführt ist.

19. Einweg-Endoskopsystem mit einem ersten Bauteil (10) und steril einzeln verpackten zweiten Bauteilen (20), welche nach Entnahme aus ihrer sterilen Verpackung lösbar mit dem ersten Bauteil (10) koppelbar sind, um ein Endoskop nach einem der Ansprüche 1 bis 18 zu erhalten,
wobei die zweiten Bauteile (20) jeweils ein mit dem ersten Bauteil (10) verbindbares proximales Ende (22) und ein distales Ende (21) aufweisen, wobei im distalen Ende (21) ein Element zur Bildaufnahme angeordnet ist, und wobei in den zweiten Bauteilen (20) jeweils ein Lichtleiter (26) mit zumindest einer lichtleitenden Faser (27) verläuft, um Licht der Lichtquelle (13) vom proximalen Ende (22) zum distalen Ende (21) zu leiten und am distalen Ende (21) abzugeben, wobei im ersten Bauteil (10) eine Lichtquelle (13) eingebaut ist, wobei die Lichtquelle (13) mindestens einen LED-Chip (14) zur Abgabe von Licht umfasst, wobei der LED-Chip (14) so mit dem am ersten Bauteil (10) verbindbaren proximalen Ende (22) eines zweiten Bauteils (20) koppelbar ist, dass das vom LED-Chip (14) abgegebene Licht in die zumindest eine lichtleitende Faser (27) des Lichtleiters (26) einkoppelbar ist und wobei die mindestens eine lichtleitende Faser (27) einen Durchmesser von mindestens 80 µm aufweist und wobei die mindestens eine lichtleitende Faser (27) eine zumindest teil- und/oder abschnittsweise an der Mantelfläche angeordnete polymerbasierte Beschichtung und/oder eine Schutzhülle aus einem polymerbasierten Schlauchmaterial aufweist, und wobei die wenigstens eine lichtleitende Faser (27) eine Glasfaser ist.

20. Einweg-Endoskopsystem nach Anspruch 19, wobei ein zweites Bauteil (20) ausgebildet als zumindest abschnittsweise flexibler Schaft vorgesehen ist, welcher eine flexible Ummantelung mit einem Schlauch oder Geflechtschlauch oder Schrumpfschlauch umfasst, welche den Lichtleiter (26) mit seiner mindestens einen lichtleitenden Faser (27) sowie eine Versorgungsleitung zur elektrischen Speisung des Kamerachips (23) zumindest abschnittsweise umschließt.

21. Einweg-Endoskopsystem nach einem der Ansprüche 19 oder 20, wobei ein zweites Bauteil (20) ausgebildet als zumindest abschnittsweise starrer Schaft vorgesehen ist, welcher eine starre Ummantelung mit einer Hülse umfasst, welche den Lichtleiter (26) mit seiner mindestens einen lichtleitenden Faser (27) sowie eine Versorgungsleitung zur elektrischen Speisung des Kamerachips (23) umschließt.

## Claims

1. An endoscope (1) comprising a first component (10) and a second component (20);
the second component (20) having a proximal end (22) coupled to the first component (10), and a distal end (21) with an element for image capturing arranged in said distal end (21), and with an optical waveguide (26) comprising at least one light-conducting fibre (27) extending through the second component (20) for directing light of a light source (13) from the proximal end (22) to the distal end (21) and emitting said light at the distal end (21);
wherein a light source (13) is installed in the first component (10), said light source (13) comprising at least one LED chip (14) for emitting light, wherein the LED chip (14) is coupled to the proximal end (22) of the second component (20) connected to the first component (10) in such a way that the light emitted by the LED chip (14) can be injected into the at least one light-conducting fibre (27) of the optical waveguide (26);
and wherein the at least one light-conducting fibre (27) has a diameter of at least 80 µm; and wherein the at least one light-conducting fibre (27) has a polymer-based coating and/or a protective cover made of a polymer-based tubing material arranged at least partially on its lateral surface and/or in sections thereof;
**characterized in that**
the at least one light-conducting fibre (27) is a glass fibre.

2. The endoscope (1) according to claim 1, wherein the optical waveguide (26) comprises not more than twenty light-conducting fibres (27).

3. The endoscope (1) according to any one of claims 1 or 2, wherein the one light-conducting fibre (27) or the plurality of light-conducting fibres (27) has/have a diameter in the range from 100 µm to 1000 µm, while the individual light-conducting fibres (11) can also have diameters that differ from each other.

4. The endoscope (1) according to any one of claims 1 to 3, wherein the one light-conducting fibre (27) or the plurality of light-conducting fibres (27) are step index glass fibres.

5. The endoscope (1) according to any one of claims 1 to 4, wherein the numerical aperture (NA) in air of the one or more light-conducting fibres (27) is at least 0.7.

6. The endoscope (1) according to any one of claims 1 to 5, wherein the optical waveguide (26) comprises a plurality of light-conducting fibres (27), wherein at least one light-conducting fibre (27) has a polymer-based coating and/or a protective cover made of a polymer-based tubing material arranged at least partially on its lateral surface and/or in sections thereof.

7. The endoscope (1) according to any one of claims 1 to 6, wherein the coating comprises any of an acrylate-based, polyurethane-based, polyimide-based, epoxy-based, polyamide-based, ethylene tetrafluoroethylene copolymer-based or poly-xylylene-based compound or a mixture of one or more of these compounds or is made of such a compound or a mixture of one or more of these compounds.

8. The endoscope (1) according to any one of claims 1 to 7, wherein the coating can be applied or has been applied to the one or more light-conducting fibres (27) by dip-coating or spray-coating or extruding or deposition at low pressure.

9. The endoscope (1) according to any one of claims 1 to 8, wherein the coating has a thickness between at least 5 µm and at most 100 µm.

10. The endoscope according to any one of claims 1 to 9, having a further outer coating which preferably comprises PMMA, polyamide (NYLON), polyimide or a fluorinated polymer or a thermoplastic elastomer and/or polyvinylidene fluoride or polytetrafluoroethylene or else polyurethane, or mixtures thereof.

11. The endoscope (1) according to any one of claims 1 to 10, wherein at the proximal end (22) of the second component, the one light-conducting fibre (27) or the plurality of light-conducting fibres (27) are arranged in an optical plug-in connector (28) in the form of a coupling sleeve.

12. The endoscope (1) according to any one of claims 1 to 11, wherein the light-conducting fibres (27) are arranged in a hot-fused form at the proximal end (22).

13. The endoscope according to any one of claims 1 to 12, wherein the at least one light-conducting fibre (27) and/or the plurality of light-conducting fibres (27) and/or the optical waveguide (26) is/are deformed at the distal end (21) compared to the proximal end (22).

14. The endoscope according to any one of claims 1 to 13, wherein at least at the distal end (21) of the optical waveguide (26) at least one light-conducting fibre (27) and/or the plurality of light-conducting fibres (27) has/have a cross section with a flattened shape having an aspect ratio of at least 1.5:1 and/or an oval cross-sectional profile and/or a kidney-shaped cross-sectional profile and/or a cross-sectional profile which is delimited by at least two lines that have different radii of curvature and/or which is defined by a differential area of two only partially overlapping circles and/or ellipses.

15. The endoscope (1) according to any one of claims 1 to 14, wherein the light source (13) can be designed to be integrated into the plug-in connector (30) and/or can have a heat sink (35) and/or an integrated LED driver circuit (32); and wherein the integrated LED (31) or the permanently integrated driver circuit (32) is connectable to the first component (10) of the endoscope (1) via electrical terminals (34).

16. The endoscope (1) according to any one of claims 1 to 15, wherein the light coupling between the integrated LED (31) and the at least one light-conducting fibre (27) of the optical waveguide (26) is in the form of a butt coupling, wherein the at least one light-conducting fibre (27) is directly glued to the chip of the integrated LED (31) using a transparent adhesive.

17. The endoscope (1) according to any one of claims 1 to 16, further comprising a housing (41) which has a seat for the camera chip (23) for positioning the camera chip (23).

18. The endoscope (1) according to claim 17, wherein the housing (41) is designed so as to comprise an opaque or dark coloured material or is made of an opaque or dark coloured material.

19. A disposable endoscope system comprising a first component (10) and second components (20) which are individually packaged in a sterile manner and which, once removed from their sterile package, can be detachably coupled to the first component (10) in order to obtain an endoscope according to any one of claims 1 to 18;
wherein each of the second components (20) has a proximal end (22) that can be coupled to the first component (10), and a distal end (21) with an element for image capturing arranged in said distal end (21), and with an optical waveguide (26) comprising at least one light-conducting fibre (27) extending through each of the second components (20) for directing light of the light source (13) from the proximal end (22) to the distal end (21) and emitting the light at the distal end (21); wherein the first component (10) has a light source (13) integrated therein, said light source (13) comprising at least one LED chip (14) for emitting light; wherein the LED chip (14) can be coupled to the proximal end (22) of a second component (20) which can be coupled to the first component (10) in such a way that the light emitted by the LED chip (14) can be injected into the at least one light-conducting fibre (27) of the optical waveguide (26); and wherein the at least one light-conducting fibre (27) has a diameter of at least 80 µm; and wherein the at least one light-conducting fibre (27) has a polymer-based coating and/or a protective cover made of a polymer-based tubing material arranged at least partially on its lateral surface and/or in sections thereof; and wherein the at least one light-conducting fibre (27) is a glass fibre.

20. The disposable endoscope system according to claim 19, wherein a second component (20) is provided in the form of a shaft which is flexible at least in sections thereof and which comprises a flexible jacket comprising a tubing or braided tubing or shrink tubing, which at least partially encloses the optical waveguide (26) including the at least one light-conducting fibre (27) thereof as well as a power supply line for electrically powering the camera chip (23).

21. The disposable endoscope system according to any one of claims 19 or 20, wherein a second component (20) is provided in the form of a shaft which is rigid at least in sections thereof and which comprises a rigid jacket comprising a sleeve which encloses the optical waveguide (26) including the at least one light-conducting fibre (27) thereof as well as a power supply line for electrically powering the camera chip (23).

## Revendications

1. Endoscope (1), comprenant un premier composant (10) et un deuxième composant (20), dans lequel le deuxième composant (20) présente une extrémité proximale (22), reliée au premier composant (10), et une extrémité distale (21), dans lequel un élément de prise de vue est disposé dans l'extrémité distale (21), et dans lequel un guide de lumière (26), doté d'au moins une fibre guidant la lumière (27), s'étend dans le deuxième composant (20) aux fins de guider la lumière de la source lumineuse (13) depuis l'extrémité proximale (22) jusqu'à l'extrémité distale (21) et la délivrer à l'extrémité distale (21),
- dans lequel une source lumineuse (13) est installée dans le premier composant (10), la source lumineuse (13) comportant au moins une puce à LED (14) destinée à émettre de la lumière, la puce à LED (14) étant couplée à l'extrémité proximale (22) du deuxième composant (20), reliée au premier composant (10), de manière à ce que la lumière émise par la puce à LED (14) puisse être injectée dans la fibre guidant la lumière (27), au nombre d'au moins une, du guide de lumière (26) ; et dans lequel la fibre guidant la lumière (27), au nombre d'au moins une, présente un diamètre d'au moins 80 µm, et dans lequel la fibre guidant la lumière (27), au nombre d'au moins une, présente un revêtement à base de polymère, appliqué au moins partiellement et/ou par portions sur la surface de la gaine, et/ou une enveloppe de protection constituée d'un matériau de tube à base de polymère,
**caractérisé en ce que** la fibre guidant la lumière (27), au nombre d'au moins une, est une fibre de verre.

2. Endoscope (1) selon la revendication 1, dans lequel le guide de lumière (26) présente au maximum vingt fibres guidant la lumière (27).

3. Endoscope (1) selon une des revendications 1 ou 2, dans lequel l'unique fibre guidant la lumière (27) ou la pluralité de fibres guidant la lumière (27) présentent un diamètre qui est compris dans la plage allant de 100 µm à 1 000 µm, les fibres individuelles guidant la lumière (27) pouvant également présenter des diamètres différents.

4. Endoscope (1) selon une des revendications 1 à 3, dans lequel l'unique fibre guidant la lumière (27) ou la pluralité de fibres guidant la lumière (27) sont des fibres de verre à saut d'indice.

5. Endoscope (1) selon une des revendications 1 à 4, dans lequel l'ouverture numérique (NA) vis-à-vis de l'air de la fibre guidant la lumière (27) ou de la pluralité de fibres guidant la lumière (27) est au moins de 0,7.

6. Endoscope (1) selon une des revendications 1 à 5, dans lequel le guide de lumière (26) comprend plusieurs fibres guidant la lumière (27), où au moins une fibre guidant la lumière (27) présente un revêtement à base de polymère, appliqué au moins partiellement et/ou par portions sur la surface de la gaine de celle-ci, et/ou une enveloppe de protection constituée d'un matériau de tube à base de polymère.

7. Endoscope (1) selon une des revendications 1 à 6, dans lequel le revêtement comporte un composé à base d'acrylate, polyuréthane, polyimide, époxy, polyamide, copolymère d'éthylène tétrafluoroéthylène ou polyxylylène ou un mélange d'un ou plusieurs de ces composés ou est constitué d'un composé de ce type ou d'un mélange d'un ou plusieurs de ces composés.

8. Endoscope (1) selon une des revendications 1 à 7, dans lequel le revêtement peut être appliqué ou est appliqué en présence d'une faible pression sur la ou les fibres guidant la lumière (27), par immersion, pulvérisation, extrusion ou dépôt.

9. Endoscope (1) selon une des revendications 1 à 8, dans lequel le revêtement présente une épaisseur comprise entre au moins 5 µm et au maximum 100 µm.

10. Endoscope (1) selon une des revendications 1 à 9, présentant un revêtement extérieur supplémentaire qui comporte de préférence du PMMA, du polyamide (NYLON), du polyimide ou un polymère fluoré ou un élastomère thermoplastique et/ou du polyfluorure de vinylidène ou du polytétrafluoroéthylène ou bien également du polyuréthane ou des mélanges de ceux-ci.

11. Endoscope (1) selon une des revendications 1 à 10, dans lequel l'unique fibre guidant la lumière (27) ou la pluralité de fibres guidant la lumière (27) sont disposées, à l'extrémité proximale (22) du deuxième composant, dans un connecteur enfichable (28) optique réalisé sous forme de manchon de couplage.

12. Endoscope (1) selon une des revendications 1 à 11, dans lequel les fibres guidant la lumière (27) sont disposées par fusion à chaud à l'extrémité proximale (22).

13. Endoscope (1) selon une des revendications 1 à 12, dans lequel la fibre guidant la lumière (27), au nombre d'au moins une, et/ou la pluralité de fibres guidant la lumière (27) et/ou le guide de lumière (26) sont déformés à l'extrémité distale (21) par rapport à l'extrémité proximale (22).

14. Endoscope (1) selon une des revendications 1 à 13, dans lequel au moins une fibre guidant la lumière (27) et/ou la pluralité de fibres guidant la lumière (27) présentent, au moins à l'extrémité distale (21) du guide de lumière (26), une section transversale de forme aplatie, avec un rapport d'aspect d'au moins 1,5 : 1, et/ou une surface de section transversale ovale et/ou une surface de section transversale réniforme et/ou une surface de section transversale qui est délimitée par au moins deux lignes ayant des rayons de courbure différents l'une de l'autre, et/ou qui est réalisée sous forme de surface différentielle de deux cercles et/ou ellipses se chevauchant seulement de manière partielle.

15. Endoscope (1) selon une des revendications 1 à 14, dans lequel la source lumineuse (13) peut être réalisée sous une forme montée dans le connecteur enfichable (30) et/ou peut présenter un dissipateur de chaleur (35) et/ou un circuit d'attaque de LED (32) intégré, et dans lequel la LED (31) intégrée ou le circuit d'attaque (32) installé de manière fixe peuvent être reliés au premier composant (10) de l'endoscope (1) à l'aide de contacts électriques (34).

16. Endoscope (1) selon une des revendications 1 à 15, dans lequel le couplage de lumière entre la LED (31) intégrée et la fibre guidant la lumière (27), au nombre d'au moins une, du guide de lumière (26) est réalisé comme couplage abouté, la fibre guidant la lumière (27), au nombre d'au moins une, étant collée directement sur la puce de la LED (31) intégrée, à l'aide d'un adhésif transparent.

17. Endoscope (1) selon une des revendications 1 à 16, comprenant en outre une enceinte (41) qui présente un logement pour la puce de caméra (23), aux fins de positionner la puce de caméra (23).

18. Endoscope (1) selon la revendication 17, dans lequel l'enceinte (41) est réalisée de manière à ce qu'elle comporte un matériau opaque ou teinté d'une couleur foncée ou est réalisée à partir d'un matériau opaque ou teinté d'une couleur foncée.

19. Système d'endoscope à usage unique, comprenant un premier composant (10) et des deuxièmes composants (20) à emballage stérile individuel qui, après leur retrait de leur emballage stérile, peuvent être accouplés de façon amovible au premier composant (10) pour obtenir un endoscope selon une des revendications 1 à 18, dans lequel les deuxièmes composants (20) présentent chacun une extrémité proximale (22), pouvant être reliée au premier composant (10), et une extrémité distale (21) ; dans lequel un élément de prise de vue est disposé dans l'extrémité distale (21) ; et dans lequel un guide de lumière (26), doté d'au moins une fibre guidant la lumière (27), s'étend dans les deuxièmes composants (20) aux fins de guider la lumière de la source lumineuse (13) depuis l'extrémité proximale (22) jusqu'à l'extrémité distale (21) et la délivrer à l'extrémité distale (21) ; dans lequel une source lumineuse (13) est installée dans le premier composant (10), la source lumineuse comportant au moins une puce à LED (14) destinée à émettre de la lumière, la puce à LED (14) pouvant être couplée à l'extrémité proximale (22) d'un deuxième composant (20), susceptible d'être reliée au premier composant (10), de manière à ce que la lumière émise par la puce à LED (14) puisse être injectée dans la fibre guidant la lumière (27), au nombre d'au moins une, du guide de lumière (26) ; et dans lequel la fibre guidant la lumière (27), au nombre d'au moins une, présente un diamètre d'au moins 80 µm ; et dans lequel la fibre guidant la lumière (27), au nombre d'au moins une, présente un revêtement à base de polymère, appliqué au moins partiellement et/ou par portions sur la surface de la gaine, et/ou une enveloppe de protection constituée d'un matériau de tube à base de polymère ; et dans lequel la fibre guidant la lumière (27), au nombre d'au moins une, est une fibre de verre.

20. Système d'endoscope à usage unique selon la revendication 19, dans lequel il est prévu un deuxième composant (20) réalisé sous la forme d'une tige qui est souple au moins par portions et qui comprend une enveloppe souple avec un tube ou un tube tressé ou un tube thermorétractable, qui entoure au moins par portions le guide de lumière (26), avec sa fibre guidant la lumière (27), au nombre d'au moins une, ainsi qu'une ligne d'alimentation pour l'alimentation électrique de la puce de caméra (23).

21. Système d'endoscope à usage unique selon une des revendications 19 ou 20, dans lequel il est prévu un deuxième composant (20) réalisé sous la forme d'une tige qui est rigide au moins par portions et qui comprend une enveloppe rigide avec un manchon qui entoure le guide de lumière (26), avec sa fibre guidant la lumière (27), au nombre d'au moins une, ainsi qu'une ligne d'alimentation pour l'alimentation électrique de la puce de caméra (23).
